# FASCICULE DE BREVET EUROPEEN

(11) **EP 2 874 998 B1**
(45) Date de publication et mention de la délivrance du brevet: **12.09.2018**
(21) Numéro de dépôt: 13735285.2
(22) Date de dépôt: 11.07.2013
(51) Int. Cl.: C07C 303/02, C07C 303/38, C07C 303/44, C07C 311/48

(54) **PROCÉDÉ DE PRÉPARATION D'UN COMPOSÉ SULFONIMIDE ET DE SES SELS**
VERFAHREN ZUR HERSTELLUNG EINER SULFONIMIDVERBINDUNG UND SALZE DAVON
METHOD FOR PREPARING A SULFONIMIDE COMPOUND AND SALTS THEREOF

(30) Priorité: 23.07.2012 FR 1257126
(43) Date de publication de la demande: 27.05.2015
(73) Titulaire: Rhodia Opérations, 75009 Paris (FR)
(72) Inventeur: BESSON, Bernard, F-01700 Miribel-les Echets (FR); METZ, François, F-69540 Irigny (FR); BUISINE, Olivier, F-69230 Saint-Genis-Laval (FR)
(74) Mandataire: Menville, Laure
(86) Numéro de dépôt international: PCT/EP2013/064652
(87) Numéro de publication internationale: WO 2014/016130

(56) Documents cités:
- EP-A1- 0 735 023
- EP-A1- 1 029 850
- WO-A1-97/23448
- DE-A1- 2 239 817
- US-A- 2 732 398
- FOROPOULOS J ET AL: "SYNTHESIS, PROPERTIES, AND REACTIONS OF BIS((TRIFUOROMETHYL)SULFONYL) IMIDE, (CF3SO2)2NH1", INORGANIC CHEMISTRY, AMERICAN CHEMICAL SOCIETY, EASTON, US, vol. 23, no. 23, 7 novembre 1984 (1984-11-07), pages 3720-3723, XP000196672, ISSN: 0020-1669, DOI: 10.1021/IC00191A011
- FOROPOULOS J ET AL: "SYNTHESIS, PROPERTIES, AND REACTIONS OF BIS((TRIFUOROMETHYL)SULFONYL) IMIDE, (CF3SO2)2NH1", INORGANIC CHEMISTRY, AMERICAN CHEMICAL SOCIETY, EASTON, US, vol. 23, no. 23, 7 November 1984 (1984-11-07), pages 3720-3723, XP000196672, ISSN: 0020-1669, DOI: 10.1021/IC00191A011

## Description

La présente invention concerne un procédé de préparation d'un composé sulfonimide et de ses sels, notamment de ses sels de lithium.

Les sels de sulfonimides, tels que le bis(trifluorométhanesulfonyl)imidure de lithium ((CF₃SO₂)₂NLi ou LiTFSI) ou le bis(perfluoroéthanesulfonyl)imidure de lithium (C₂F₅SO₂)₂NLi), sont des composés d'intérêt particulier. Ils possèdent notamment des propriétés qui en font des composés de valeur pour les applications électroniques exigeantes du point de vue pureté et qualité, par exemple la conduction et/ou la dissipation de charges électroniques dans les marchés batteries ou antistatiques. Ces propriétés qui sont, d'une part, une acidité très élevée de l'imide, et d'autre part, une absence de pouvoir complexant de l'imidure vis-à-vis des différents cations. Ces mêmes propriétés rendent extrêmement difficiles et délicates les opérations de purification. La forte acidité implique une forte dissociation et une forte dissociation implique une solubilité élevée dans les milieux polaires tels que l'eau. De plus, ces imidures forment souvent des composés d'addition avec l'eau, et sont de ce fait difficiles à séparer de l'eau. De ce fait, il est préférable que les sels de sulfonimide, tels que (CF₃SO₂)₂NLi ou ((C₂F₅SO₂)₂NLi), présentent une pureté élevée, qu'ils soient sous forme anhydre ou sous forme de solution aqueuse.

Il existe dans l'état de la technique des procédés de préparation de sels de sulfonimide comprenant notamment une étape de purification du sel de sulfonimide par recristallisation dans des solvants tels que le dioxane. Or cette étape de purification présente plusieurs inconvénients, tels que l'utilisation de solvant toxique et inflammable, la difficulté de séparer le sel de sulfonimide des complexes d'association avec le dioxane, l'obtention d'une pureté limitée. De plus, il a été observé que de tels procédés conduisent souvent à des sels colorés, présentant donc une pureté qui n'est pas optimale. La demande de brevet EP 1 029 850 décrit un procédé de préparation de sulfonimide ou de ses sels comprenant la cristallisation du sel de métal alcalin de sulfonimide dans un milieu aqueux. Par ailleurs, il existe également des procédés de préparation de sels de sulfonimide, dont l'étape de purification du sel résultant consiste en des extractions liquide/liquide. Or, en général les extractions de ce type ne sont guère purifiantes et impliquent l'utilisation de larges quantités de solvant.

Par ailleurs, les sels de sulfonimide sont généralement préparés à partir de sulfonimide. Toutefois, les sulfonimides sont typiquement obtenus sous la forme d'un mélange avec des impuretés organiques telles que les acides sulfinique, sulfonique ou des sulfonamides. Or, ces impuretés sont difficilement séparables du sulfonimide. Il en résulte que les sels de sulfonimide résultant ne présentent pas une pureté optimale.

Un mode de purification d'un mélange de trifluorométhylsulfinimide avec des impuretés sulfoniques est déjà connu par cristallisation/sublimation de Foropoulos et al., Inorganic Chemistry, 23(23), 3720-3723 (1984).

Au vu de l'importance technologiques des sels de sulfonimide, et notamment du (CF₃SO₂)₂NLi, il existe un besoin de mettre au point un procédé de préparation facile à mettre en oeuvre et ne présentant pas les inconvénients mentionnés ci-dessus. Cependant, au vu de l'absence de connaissance des différentes interactions entre les impuretés organiques en mélange dans l'eau avec les sulfonimides, il est particulièrement difficile de mettre au point une étape de purification efficace desdits sulfonimides. Il existe donc un besoin de mettre au point un procédé de préparation de sulfonimides, notamment de (CF₃SO₂)₂NH, et des sels de sulfonimide correspondants, notamment (CF₃SO₂)₂NLi, ayant des puretés élevées.

La présente invention a pour but de fournir un procédé de préparation de sulfonimide notamment de (CF₃SO₂)₂NH, de pureté élevée.

Un autre but de la présente invention consiste à fournir un procédé de préparation de sel de sulfonimide, et notamment de (CF₃SO₂)₂NLi, de pureté élevée.

Un autre but de la présente invention est de fournir un procédé de préparation de sel de sulfonimide, et notamment de (CF₃SO₂)₂NLi, facile à mettre en oeuvre.

### Procédé de préparation de (Rf¹SO₂)(Rf²SO₂)NH aqueux

La présente invention a pour objet un procédé de préparation d'un composé sulfonimide de formule (Rf¹-SO₂)(Rf²-SO₂)NH aqueux, Rf¹ et Rf² étant indépendamment l'un de l'autre choisi dans le groupe constitué de : l'atome de fluor et des groupes ayant de 1 à 10 atomes de carbone étant choisis parmi les groupes perfluoroalkyles, et fluoroalkyles, à partir d'un mélange M1 comprenant (Rf¹-SO₂)(Rf²-SO₂)NH, Rf¹SO₂H et/ou Rf²SO₂H, et caractérisé en ce qu'il comprend une étape d'oxydation dudit mélange M1 par un agent oxydant, pour obtenir un mélange M2 comprenant (Rf¹-SO₂)(Rf²-SO₂)NH, Rf¹SO₃H et/ou Rf²SO₃H, et ii) une étape de distillation du mélange M2, en milieu aqueux pour séparer (Rf¹SO₂)(Rf²SO₂)NH de Rf¹SO₃H et/ou Rf²SO₃H.

Selon l'invention, Rf¹ et Rf² peuvent être identiques ou différents.

Selon un mode de réalisation, Rf¹ et Rf² sont indépendamment l'un de l'autre choisi dans le groupe constitué de : l'atome de fluor et des groupes ayant de 1 à 10 atomes de carbone étant choisis parmi les groupes perfluoroalkyles et fluoroalkyles.

Cette étape d'oxydation permet de manière surprenante d'oxyder sélectivement Rf¹SO₂H et/ou Rf²SO₂H en Rf¹SO₃H et/ou Rf²SO₃H.

Selon l'invention, le mélange M2 peut être soumis avantageusement à une étape de distillation en milieu aqueux pour séparer (Rf¹-SO₂)(Rf²-SO₂)NH de Rf¹SO₃H et/ou de Rf²SO₃H, et de Rf¹SO₂NH₂ et/ou Rf²SO₂NH₂. Ainsi, la pureté du (Rf¹-SO₂)(Rf²-SO₂)NH obtenu est élevée.

Selon un mode de réalisation, le mélange M1 comprend (Rf¹-SO₂)(Rf²-SO₂)NH, Rf¹SO₂H et/ou Rf²SO₂H, Rf¹SO₂NH₂ et/ou Rf²SO₂NH₂.

Conformément à l'invention, le composé sulfonimide de formule (Rf¹SO₂)(Rf²SO₂)NH obtenu à l'issue de la mise en oeuvre du procédé selon l'invention est présent en solution aqueuse.

Selon un mode de réalisation, lorsque Rf¹ et Rf² sont identiques, le composé sulfonimide (Rf¹SO₂)(Rf²SO₂)NH synthétisé est un sulfonimide dit « symétrique ». De manière préférée, ledit sulfonimide symétrique est choisi dans le groupe constitué de : (CF₃SO₂)₂NH, (CHF₂SO₂)₂NH, (CH₂FSO₂)₂NH, (C₂F₅SO₂)₂NH, (C₃F₇SO₂)₂NH, F(SO₂)₂NH et (C₄F₉SO₂)₂NH.

Selon un mode de réalisation, lorsque Rf¹ et Rf² sont différents, le composé sulfonimide (Rf¹SO₂)(Rf²SO₂)NH synthétisé est choisi dans le groupe constitué de : (FSO₂)(CF₃SO₂)NH, (FSO₂)(C₂F₅SO₂)NH, (FSO₂)(C₃F₇SO₂)NH, (FSO₂)(C₄F₉SO₂)NH, (CF₃SO₂)(C₂F₅SO₂)NH, (CF₃SO₂)(C₃F₇SO₂)NH, (CF₃SO₂)(C₄F₉SO₂)NH, (C₂F₅SO₂)(C₃F₇SO₂)NH, (C₂F₅SO₂)(C₄F₉SO₂)NH, et (C₃F₇SO₂)(C₄F₉SO₂)NH.

Dans le cadre de l'invention, et sauf mention contraire, lorsque Rf¹ et Rf² sont identiques, le sulfonimide (ou ses sels) obtenu est un sulfonimide (ou ses sels) symétrique.

Dans le cadre de l'invention, et sauf mention contraire, lorsque Rf¹ et Rf² sont différents, le sulfonimide (ou ses sels) obtenu est un sulfonimide (ou ses sels) dissymétrique.

Selon un mode de réalisation, Rf¹ et Rf² sont identiques.

Selon un mode de réalisation, Rf¹ et Rf² sont des groupes perfluoroalkyles comprenant de 1 à 10 atomes de carbone, de préférence de 1 à 4, et encore plus préfârentiellement de 1 à 2 atomes de carbone.

Selon un mode de réalisation, Rf¹ et Rf² représentent CF₃. Dans ce cas, le procédé susmentionné permet de préparer (CF₃SO₂)₂NH aqueux, c'est-à-dire une solution aqueuse de (CF₃SO₂)₂NH.

Selon un autre mode de réalisation, Rf¹ et Rf² représentent C₂F₅. Dans ce cas, le procédé susmentionné permet de préparer (C₂F₅SO₂)₂NH aqueux, c'est-à-dire une solution aqueuse de (C₂F₅SO₂)₂NH.

Selon un mode de réalisation, les composés Rf¹SO₂H et Rf²SO₂H sont indépendamment l'un de l'autre, choisis parmi le groupe constitué de : CHF₂SO₂H, CH₂FSO₂H, CF₃SO₂H, C₂F₅SO₂H, C₃F₇SO₂H, FSO₂Het C₄F₉SO₂H.

Selon un mode de réalisation, les composés Rf¹SO₃H et Rf²SO₃H sont indépendamment l'un de l'autre, choisis parmi le groupe constitué de: CHF₂SO₂H, CH₂FSO₂H, CF₃SO₃H, C₂F₅SO₃H, C₃F₇SO₃H, FSO₃H et C₄F₉SO₃H.

Dans le cadre de l'invention, et sauf mention contraire, on entend par « (Rf¹SO₂)(Rf²SO₂)NH aqueux », une solution aqueuse de (Rf¹SO₂)(Rf²SO₂)NH.

Selon un mode de réalisation, Rf¹SO₂H et/ou Rf¹SO₂H, et notamment CF₃SO₂H, est un produit minoritaire dans le mélange M1 et (Rf¹SO₂)(Rf²SO₂)NH, et notamment (CF₃SO₂)₂NH, est un produit majoritaire dans ledit mélange.

Dans le cadre de l'invention, et sauf mention contraire, on entend par « produit minoritaire dans un mélange», un produit constituant au plus 20% en poids dudit mélange, de manière préférée au plus 10%, de préférence au plus 5%, par rapport aux autres constituants dudit mélange.

Dans le cadre de l'invention, et sauf mention contraire, on entend par « produit majoritaire dans un mélange», un produit constituant au moins 30% en poids dudit mélange, de préférence au moins 50%, par rapport aux autres constituants dudit mélange.

Selon un mode de réalisation, le mélange M1 comprend de 0,01% à 5%, de préférence de 0,1% à 2% en poids de Rf¹SO₂H et/ou de Rf²SO₂H.

Selon un mode de réalisation, le mélange M1 comprend de 30% à 95% en poids de (Rf¹SO₂)(Rf²SO₂)NH, et notamment (CF₃SO₂)₂NH, de préférence de 50% à 95%, et préférentiellement de 60% à 95% en poids.

Selon l'invention, le mélange M1 peut comprendre en outre d'autres composés, notamment choisis parmi Rf¹SO₂NH₂, Rf²SO₂NH₂, Rf¹SO₃H, Rf²SO₃H, HF, HBr et HCl. En particulier ces composés sont des produits secondaires minoritaires dans le mélange M1. Selon l'invention, on parle également d'impuretés organiques et minérales.

Selon un mode de réalisation, les composés Rf¹SO₂NH₂ et Rf²SO₂NH₂ sont indépendamment l'un de l'autre, choisis parmi le groupe constitué de : CHF₂SO₂NH₂, CH₂FSO₂NH₂, CF₃SO₂NH₂, C₂F₅SO₂NH₂, C₃F₇SO₂NH₂ et C₄F₉SO₂NH₂. En particulier Rf¹SO₂NH₂ et/ou Rf²SO₂NH₂ représente(nt) CF₃SO₂NH₂.

Selon un mode de réalisation, le mélange M1 comprend (Rf¹SO₂)(Rf²SO₂)NH, et notamment (CF₃SO₂)₂NH, et des impuretés organiques et minérales telles que Rf¹SO₂H, Rf²SO₃H, Rf¹SO₂NH₂, Rf²SO₂NH₂, Rf¹SO₃H, Rf²SO₃H HF, HBr ou HCl. En particulier, M1 comprend (CF₃SO₂)₂NH et des impuretés organiques et minérales choisies dans le groupe constitué de : CF₃SO₂H, CF₃SO₂NH₂, CF₃SO₃H, HF, HBr et HCl.

Selon un mode de réalisation, le mélange M1 comprend de 0,01% à 5%, de préférence de 0,1% à 2% en poids de Rf¹SO₂NH₂ et/ou de Rf²SO₂NH₂.

Dans le procédé selon l'invention, l'agent oxydant de l'étape i) d'oxydation peut être choisi dans le groupe constitué de : l'eau oxygénée, les hydroperoxydes et peroxydes organiques, l'eau de javel, le persulfate de sodium, le perborate de sodium, l'oxone, l'ozone et le protoxyde d'azote. En particulier, l'agent oxydant est l'eau oxygénée (H₂O₂), notamment H₂O₂ à 30% en poids.

Selon un mode de réalisation, l'étape d'oxydation i) est effectuée à une température allant de 20°C à 100°C, de préférence de 50°C à 90°C et préférentiellement de 75°C à 85°C. En particulier, l'étape d'oxydation i) est réalisée à 80°C.

Selon un mode de réalisation, l'étape d'oxydation i) est effectuée de 5 à 120 minutes, de préférence de 10 à 60 minutes, et préférentiellement de 20 à 40 minutes.

Selon l'invention, le mélange M2 peut comprendre en outre d'autres composés, notamment choisis parmi Rf¹SO₂NH₂, Rf²SO₂NH₂, Rf¹SO₃H, Rf²SO₃H, HF, HBr et HCl. En particulier ces composés sont des produits secondaires minoritaires dans le mélange M2. Selon l'invention, on parle également d'impuretés organiques et minérales.

Selon un mode de réalisation, le mélange M2 comprend (Rf¹SO₂)(Rf²SO₂)NH, et des impuretés organiques et minérales telles que Rf¹SO₂NH₂, Rf²SO₂NH_{2,}, Rf¹SO₃H, Rf²SO₃H, HF, HBr et HCl. En particulier, M2 comprend (CF₃SO₂)₂NH et des impuretés organiques et minérales telles que CF₃SO₂NH₂, CF₃SO₃H, HF, HBr et HCl.

Selon un mode de réalisation, le mélange M2 ne comprend pas de Rf¹SO₂H et/ou Rf²SO₂H, tel que CF₃SO₂H, ou seulement des quantités très faibles, c'est-à-dire inférieures à 200ppm, de préférence inférieures à 50ppm, et de préférence inférieures à 10 ppm.

Selon un mode de réalisation, le mélange M2 comprend de 0,01% à 5%, de préférence de 0.1% à 2% en poids de Rf¹SO₂NH₂ et/ou de Rf²SO₂NH₂.

Il a été montré que l'étape d'oxydation du mélange M1 permet avantageusement d'oxyder Rf¹SO₂H et/ou Rf²SO₂H présent(s) dans le mélange M1 en Rf¹SO₃H et/ou Rf²SO₃H. En particulier, l'étape d'oxydation i) est une oxydation sélective de Rf¹SO₂H et/ou Rf²SO₂H par rapport à (Rf¹SO₂)(Rf²SO₂)NH et par rapport à Rf¹SO₂NH₂ et/ou Rf²SO₂NH₂. En effet, de façon surprenante Rf¹SO₂NH₂ et/ou Rf²SO₂NH₂ n'a pas été oxydé lors de l'étape i) d'oxydation.

Dans te cadre de l'invention, on entend par « oxydation sélective d'un composé par rapport à un autre», l'action d'un agent oxydant sur un composé spécifique sans affecter l'autre composé. On peut notamment citer l'oxydation sélective de Rf¹SO₂H et/ou Rf²SO₂H présent dans le mélange M1 en Rf¹SO₃H et/ou Rf²SO₃H, sans que (Rf¹SO₂)(Rf²SO₂)NH, et Rf¹SO₂NH₂ et/ou Rf²SO₂NH₂ ne soient oxydés. En particulier, on peut citer l'oxydation sélective de CF₃SO₂H présent dans le mélange M1 en CF₃SO₃H, sans que (CF₃SO₂)₂NH et CF₃SO₂NH₂ ne soient oxydés.

L'étape de distillation ii) du mélange M2 est réalisée en milieu aqueux, pour conduire à une solution aqueuse de (Rf¹SO₂)(Rf²SO₂)NH, et notamment à une solution aqueuse de (CF₃SO₂)₂NH.

Selon l'invention, l'étape de distillation ii) peut être effectuée en présence d'une quantité suffisante d'eau pour réaliser la distillation de (Rf¹SO₂)(Rf²SO₂)NH. En particulier, l'eau du milieu aqueux de l'étape ii) a été ajoutée à l'issue de l'étape d'oxydation i). En particulier, l'eau du milieu aqueux de l'étape ii) provient également de l'étape d'oxydation préalable, et notamment de l'eau oxygénée à 30% en poids.

Selon un mode de réalisation, une solution aqueuse comprenant de 40% à 95% en poids, de préférence de 60% à 80% en poids, et préférentiellement 65% à 75% en poids de (Rf¹SO₂)(Rf²SO₂)NH est obtenue à l'issue de l'étape d'oxydation i).

Selon l'invention, l'étape de distillation peut être effectuée dans un appareil de distillation comprenant un bouilleur et une colonne de distillation.

Selon un mode de réalisation, l'étape de distillation est effectuée à une température dans le bouilleur allant de 50°C à 300°C, de préférence de 50°C à 200°C, et préférentiellement de 80°C à 150°C. En particulier, la température dans le bouilleur va de 80°C à 130°C. On peut par exemple effectuer l'étape de distillation à une température dans le bouilleur de 110°C à 130°C.

Dans le cadre de l'invention, et sauf mention contraire, la température dans le bouilleur correspond à la température en pied de colonne de distillation.

Selon un mode de réalisation, l'étape de distillation est effectuée dans un appareil de distillation, comprenant une colonne de distillation dont la température en tête de colonne va de 20°C à 180°C, de préférence de 40°C à 150°C, et préférentiellement de 40°C à 130°C. En particulier, la température en tête de colonne de distillation va de 40°C à 100°C. En particulier, la température en tête de colonne va de 50°C à 80°C.

Selon un mode de réalisation, l'étape de distillation ii) est réalisée à pression atmosphérique dans la colonne de distillation. Selon un autre mode de réalisation, l'étape de distillation du mélange M2 est effectuée sous vide, notamment à une pression dans la colonne allant de 1 à 1000 mbar (0,1 à 100 kPa), de préférence de 5 à 500 mbar (0,5 à 50 kPa), et préférentiellement de 5 à 200 mbar (0,5 à 20 kPa). En particulier, la pression dans la colonne va de 5 à 100 mbar, de préférence de 5 à 60mbar. En particulier, la pression va de 20 mbar à 60 mbar.

Selon un mode de réalisation, l'étape de distillation comprend la récupération de différentes fractions de distillation, lesdites fractions étant récupérées dans différentes conditions de distillation, à savoir à différentes températures de bouilleur, de tête de colonne et de pression.

Ainsi, selon un mode de réalisation particulier, une première fraction est recueillie dans les conditions suivantes : l'étape de distillation est effectuée à une température dans le bouilleur entre 70°C et 130°C, à une pression dans la colonne allant de 20 à 60 mbar et à une température en tête de colonne allant de 40°C à 100°C.

Puis, selon un autre mode de réalisation particulier, une seconde fraction est recueillie dans les conditions suivantes: l'étape de distillation est effectuée à une température en tête de colonne allant de 50°C à 80°C, à une pression dans la colonne allant de 5 à 15 mbar, et à une température dans le bouilleur allant de 90°C à 130°C.

Selon l'invention, la colonne de distillation peut être adaptée au degré de pureté souhaité, et elle peut être constituée de matériaux adaptés aux conditions acides du mélange à distiller.

Selon l'invention, l'appareil de distillation comprenant un bouilleur et une colonne de distillation peut être constitué de matériaux adaptés aux conditions acides du mélange à distiller. En particulier, l'appareil de distillation est choisi parmi les matériaux suivants : verre, téflon, graphite.

Selon l'invention, l'étape de distillation peut être effectuée au moyen d'une colonne comprenant un nombre de plateaux théoriques adapté pour permettre la distillation de (Rf¹SO₂)(Rf²SO₂)NH dans l'eau.

Selon un mode de réalisation, la distillation est effectuée au moyen d'une colonne de distillation comprenant de 2 à 40 plateaux théoriques, de préférence de 4 à 20, et préférentiellement de 5 à 15. En particulier, la distillation est effectuée au moyen d'une colonne comprenant de 7 à 14 plateaux théoriques.

Dans le cadre de l'invention, et sauf mention contraire, on entend par « plateaux théoriques », les plateaux idéaux où les pertes de chaleur sont nulles et les équilibres thermodynamiques instantanés.

Selon un mode de réalisation, l'étape de distillation ii) est réalisée à un taux de reflux allant de 1 à 50, de préférence de 1 à 40, et préférentiellement de 5 à 30. En particulier, le taux de reflux va de 5 à 15.

Dans le cadre de l'invention, et sauf mention contraire, on entend par « taux de reflux », le ratio entre le débit molaire (massique ou volumique) de reflux (fractions de vapeurs condensées renvoyées en tête de colonne) et le débit molaire (respectivement massique ou volumique) de distillat pur récupéré.

Selon le procédé de l'invention, l'étape de distillation ii) peut être réalisée en continu ou en discontinu.

Selon un mode de réalisation, l'étape de distillation du mélange M2 permet de recueillir différentes fractions selon les produits contenus dans le mélange M2. Une partie des impuretés organiques contenues dans le mélange M2 reste en pied de colonne, telles que Rf¹SO₂NH₂ et/ou Rf²SO₂NH₂ et l'hydrate de Rf¹SO₃H et/ou de Rf²SO₃H. Ainsi, à l'issue du procédé, on récupère une solution aqueuse de (Rf¹SO₂)(Rf²SO₂)NH de pureté élevée, notamment dépourvue de Rf¹SO₂H et/ou Rf²SO₂H, de Rf¹SO₃H et/ou Rf²SO₃H, et de Rf¹SO₂NH₂ et/ou Rf²SO₂NH₂.

Selon l'invention, le procédé peut comprendre une étape de recyclage de Rf¹SO₂NH₂ et/ou Rf²SO₂NH₂.

Selon un mode de réalisation, à l'issue de l'étape de distillation ii), Rf¹SO₂NH₂ et/ou Rf²SO₂NH₂ est(sont) soumis à diverses étapes de neutralisation et d'extractions du culot de distillation. A l'issue de ces étapes, on récupère Rf¹SO₂NH₂ et/ou Rf²SO₂HH₂ pur(s).

Selon un mode de réalisation, Rf¹SO₂NH₂ et/ou Rf²SO₂NH₂ pur(s) est(sont) recyclés dans le procédé de l'invention, et notamment dans l'étape d'ammonolyse décrite par la suite.

Dans le cadre de l'invention, et sauf mention contraire, on entend par « produit ayant une pureté élevée », un produit ayant une pureté supérieure à 95%, de préférence supérieure à 99%, et préférentiellement supérieure ou égale à 99,5%.

Selon un mode de réalisation, une solution aqueuse comprenant de 40 à 95% en poids, de préférence de 60% à 80% en poids, et préfèrentiellement 65% à 75% en poids de (Rf¹SO₂)(Rf²SO₂)NH est obtenue à l'issue des étapes d'oxydation et de distillation.

Il a donc été découvert que l'étape i) d'oxydation, suivie d'une étape ii) de distillation, du mélange M1 comprenant notamment (Rf¹SO₂)(Rf²SO₂)NH et des impuretés organiques telles que Rf¹SO₂H et/ou Rf²SO₃H, Rf¹SO₃H et/ou Rf²SO₃H, et Rf¹SO₂NH₂ et/ou Rf²SO₂NH₂, permet de purifier (Rf¹SO₂)(Rf²SO₂)NH.

Rf¹SO₂H et/ou Rf²SO₂H contenu dans le mélange M1 présente(nt) notamment un point d'ébullition très proche de celui de (Rf¹SO₂)(Rf²SO₂)NH (par exemple CF₃SO₂H a un point d'ébullition de 165°C, CF₃SO₂NH₂ a une température d'ébullition de 164°C et (CF₃SO₂)₂NH a une température d'ébullition de 167°C). De plus, Rf¹SO₂H et/ou Rf²SO₂H ne forme(nt) pas d'hydrates avec l'eau. Enfin, les dérivés sulfonamides Rf¹SO₂NH₂ et/ou Rf²SO₂NH₂ sont des impuretés typiquement présentes dans les procédés de préparation de sulfonimides, et de leurs sels, qui sont difficilement séparables desdits sulfonomides.

Ainsi, l'ensemble de ces différents éléments implique que la purification de (Rf¹SO₂)(Rf²SO₂)NH est difficile.

L'étape d'oxydation selon l'invention permet notamment d'oxyder sélectivement Rf¹SO₂H et/ou Rf²SO₂H en Rf¹SO₃H et/ou Rf²SO₃H, qui peu(ven)t être ensuite séparé(s) de manière avantageuse de (Rf¹SO₂)(Rf²SO₂)NH, lors de l'étape de distillation.

De manière surprenante, cette étape d'oxydation ne permet pas d'éliminer les impuretés de type sulfonamide Rf¹SO₂NH₂ et/ou Rf²SO₂NH₂, qui ne sont pas oxydées.

La combinaison de différents paramètres spécifiques de distillation, tels que le taux de reflux, le nombre de plateaux théoriques, la température du bouilleur, la température en tête de colonne et la pression dans la colonne de distillation, permet de séparer de façon particulièrement efficace les impuretés sulfonamides Rf¹SO₂NH₂ et/ou Rf²SO₂NH₂ du produit (Rf¹SO₂)(Rf²SO₂)NH. Ainsi, une solution aqueuse de (Rf¹SO₂)(Rf²SO₂)NH de très haute pureté est avantageusement obtenue.

Par ailleurs, il a été montré que les impuretés sulfonamides Rf¹SO₂NH₂ et/ou Rf²SO₂NH₂ peuvent avantageusement être recyclées et réutilisées dans le procédé de l'invention, par exemple lors de l'introduction de sulfonamides comme réactifs de réaction. Il en découle ainsi un procédé comprenant avantageusement une étape de valorisation des impuretés sulfonamides Rf¹SO₂NH₂ et/ou Rf²SO₂NH₂, au moins par recyclage au sein du procédé. Ce recyclage est particulièrement avantageux d'un point de vue industriel.

### Etape préalable de distillation

Selon l'invention, le procédé de préparation de (Rf¹SO₂)(Rf²SO₂)NH aqueux peut comprendre une étape préalable de distillation d'un mélange M'1, comprenant (Rf¹SO₂)(Rf²SO₂)NH et Rf¹SO₂H et/ou Rf²SO₂H, conduisant au mélange M1. Typiquement, cette étape de distillation conduisant au mélange M1 est une distillation permettant de purifier grossièrement le mélange M'1. En particulier, le procédé de préparation de (CF₃SO₂)₂NH aqueux comprend une étape préalable de distillation d'un mélange M'1, comprenant (CF₂SO₂)₂NH et CF₃SO₂H, conduisant au mélange M1.

Selon un mode de réalisation, la distillation du mélange M'1 peut permettre de séparer certaines impuretés, telles que les hydrogénosulfates d'amines tertiaires NR'₃, R'₃ représentant un groupe alkyle linéaire ou branché contenant de 1 à 20 atomes de carbone, de (Rf¹SO₂)(Rf²SO₂)NH. En particulier, les hydrogénosulfates d'amines tertiaires sont les hydrogénosulfates de triéthylamine ou de diisopropyléthylamine. Ainsi, le mélange M1 correspond au mélange M'1 dépourvu notamment d'hydrogénosuffate d'amines tertiaires NR'₃.

Ainsi, la présente invention concerne un procédé de préparation de (Rf¹SO₂)(Rf²SO₂)NH aqueux, à partir d'un mélange M'1 comprenant (Rf¹SO₂)(Rf²SO₂)NH et Rf¹SO₂H et/ou Rf²SO₂H, caractérisé en ce qu'il comprend :
- une étape de distillation dudit mélange M'1, pour conduire à un mélange M1 ;
- une étape d'oxydation dudlt mélange M1 par un agent oxydant, pour obtenir un mélange M2 comprenant (Rf¹SO₂)(Rf²SO₂)NH et Rf¹SO₃H et/ou Rf²SO₂H ; et
- une étape de distillation du mélange M2, en milieu aqueux, permettant de séparer (Rf¹SO₂)(Rf²SO₂)NH et Rf¹SO₃H et/ou Rf²SO₃H.

### Autres étapes préalables

Selon un mode de réalisation, le sulfonimide (Rf¹SO₂)(Rf²SO₂)NH présent dans le mélange M'1 susmentionné est obtenu par une étape d'acidification d'une phase organique comprenant un complexe (Rf¹SO₂)(Rf²SO₂)NH,NR'₃, R' représentant un groupe alkyle, linéaire ou branché, contenant de 1 à 20 atomes de carbone. De préférence, le complexe (Rf¹SO₂)(Rf²SO₂)NH,NR'₃ est (Rf¹SO₂)(Rf²SO₂)NH,NEt₃, où Et représente le radical éthyle C₂H₅, ou (Rf¹SO₂)(Rf²SO₂)NH,NEt(i-Pr)₂, où i-Pr représente le radical isopropyle. Encore plus préférentiellement, le complexe (Rf¹SO₂)(Rf²SO₂)NH,NR'₃ est (CF₃SO₂)₂NH,NEt₃ ou (CF₃SO₂)₂NH,NEt(i-Pr)₂.

Selon un mode de réalisation, le complexe (Rf¹SO₂)(Rf²SO₂)NH,NR'₃ susmentionné est obtenu par une étape d'ammonolyse de Rf¹SO₂X et de Rf²SO₂X, X représentant Cl, Br ou F, Rf et Rf² pouvant être identiques ou différents.

Selon un autre mode de réalisation, le complexe (Rf¹SO₂)(Rf²SO₂)NH,NR'₃ susmentionné est obtenu par une étape d'ammonolyse de Rf¹SO₂X, en présence de Rf²SO₂NH₂, X représentant Cl, Br ou F, Rf¹ et Rf² pouvant être identiques ou différents.

Selon un mode de réalisation, Rf¹SO₂X est obtenu par une étape d'oxydation d'un mélange M3 comprenant Rf¹SO₂M^{a}. M^{a} représentant un métal alcalin, et Rf¹SO₂M^{a}, pour obtenir un mélange biphasique M4, ledit mélange M4 comprenant Rf¹SO₂X et Rf¹SO₂M^{a}.

Selon l'invention, M^{a} représente un métal alcalin notamment choisi parmi : K, Li, Na, Cs.

Selon un autre mode de réalisation, Rf¹SO₂X, avec X = F, est obtenu par électrofluoration de R^{h}SO₂F, R^{h} représentant une chaîne hydrocarbonée identique à celle de Rf¹, ladite chaîne comprenant des atomes d'hydrogène à la place des atomes de fluor, et R^{h}SO₂F étant éventuellement obtenu à partir de R^{h}SO₂W, avec W = Cl, Br ou I.

De préférence, W représente Cl.

Selon un mode de réalisation, le mélange M3 est obtenu par :
- une étape de sulfination de Rf¹SO₂M^{a}, notamment de CF₃CO₂K, dans un solvant organique, pour conduire à un mélange M comprenant Rf¹SO₂M^{a}, notamment CF₃SO₂K. et ledit solvant organique ; puis
- une étape de distillation dudit solvant organique du mélange M, pour obtenir le mélange M sans ledit solvant; puis
- une étape de séparation des sels, et notamment une étape d'extraction liquide/liquide, du mélange M issu de l'étape de distillation.

Selon un mode de réalisation, Rf¹CO₂M^{a} est obtenu par une étape de salification de Rf¹CO₂H, de préférence de CF₃CO₂H.

Avantageusement, au moins pour l'étape de sulfination, M^{a} est le potassium.

Ces différentes étapes sont détaillées plus précisément dans la suite de la description.

### Procédé de préparation du sel de sulfonimide (Rf¹SO₂)(Rf²SO₂)NM^{b}

La présente invention a également pour objet la préparation de (Rf¹SO₂)(Rf²SO₂)NM^{b}, M^{b} représentant un métal alcalin, notamment choisi parmi : K, Li, Na et Cs, à partir du procédé de préparation de (Rf¹SO₂)(Rf²SO₂)NH tel que décrit ci-dessus.

La présente invention a également pour objet un procédé de préparation de (Rf¹SO₂)(Rf²SO₂)NM^{b}, de préférence de (Rf¹SO₂)(Rf²SO₂)NLi, à partir de (Rf¹SO₂)(Rf²SO₂)NH aqueux, ledit procédé comprenant la préparation de (Rf¹SO₂)(Rf²SO₂)NH aqueux selon le procédé tel que décrit ci-dessus.

Selon un mode de réalisation, M^{b} représente Li. Ainsi, le procédé de préparation de (Rf¹SO₂)(Rf²SO₂)NM^{b}, correspond au procédé de préparation de (Rf¹SO₂)(Rf²SO₂)NLi.

Selon un mode de réalisation, lorsque Rf¹ et Rf² sont identiques, le sel de sulfonimide résultant est dit symétrique, et est notamment choisi parmi : (CF₃SO₂)₂NM^{b}, , (CHF₂SO₂)₂NM^{b}, (CH₂FSO₂)₂NM^{b} (C₂F₅SO₂)₂NM^{b}, (C₃F₇SO₂)₂NM^{b}, (FSO₂)₂NM^{b}, (C₄F₉SO₂)₂NM^{b}. En particulier, les sels de sulfonimide sont (FSO₂)₂NLi (Rf¹ = Rf² = F) ou (CF₃SO₂)₂NLi (Rf¹ = Rf² = CF₃) ou (C₂F₅SO₂)₂NLi (Rf¹ = Rf² = C₂F₅), et de préférence (CF₃SO₂)₂NLi et (C₂F₅SO₂)₂NLi.

Selon un mode de réalisation, lorsque Rf¹ et Rf² sont différents, le sel de sulfonimide résultant est dit dissymétrique, et est notamment choisi parmi : (FSO₂)(CF₃SO₂)NM^{b}, (FSO₂)(C₂F₅SO₂)NM^{b}, (FSO₂)(C₃F₇SO₂)NM^{b}, (FSO₂)(C₄F₉SO₂)NM^{b}, (CF₃SO₂)(C₂F₅SO₂)NM^{b}, (CF₃SO₂)(C₃F₇SO₂)NM^{b}, (CF₃SO₂)(C₄F₉SO₂)NM^{b}, (C₂F₅SO₂)(C₃F₇SO₂)NM^{b}, (C₂F₅SO₂)(C₄F₉SO₂)NM^{b}, (C₃F₇SO₂)(C₄F₉SO₂)NM^{b}.

Selon un mode de réalisation, Rf¹ et Rf², identiques ou différents, sont des perfluoroalkyles.

Selon un mode de réalisation, (Rf¹SO₂)(Rf²SO₂)NM^{b} est choisi parmi (CF₃SO₂)₂NLi et (C₂F₅SO₂)₂NLi. De préférence, (Rf¹SO₂)(Rf²SO₂)NM^{b} est (CF₃SO₂)₂NLi.

Selon un mode de réalisation, le procédé de préparation de (Rf¹SO₂)(Rf²SO₂)NM^{b} comprend la préparation de (Rf¹SO₂)(Rf²SO₂)NH aqueux à partir d'un mélange M1 comprenant (Rf¹SO₂)(Rf²SO₂)NH et Rf¹SO₂H et/ou Rf²SO₂H selon le procédé tel que décrit ci-dessus et caractérisé en ce qu'il comprend :
i) une étape d'oxydation dudit mélange M1 par un agent oxydant, pour obtenir un mélange M2 comprenant (Rf¹SO₂)(Rf²SO₂)NH et Rf¹SO₃H et/ou Rf²SO₃H; et
ii) une étape de distillation du mélange M2, en milieu aqueux pour séparer (Rf¹SO₂)(Rf²SO₂)NH de Rf¹SO₃H et/ou de Rf²SO₃H.

Selon un mode de réalisation, le procédé de préparation de (Rf¹SO₂)(Rf²SO₂)NM^{b}, et notamment de (Rf¹SO₂)(Rf²SO₂)NLi, comprend une étape de neutralisation de (Rf¹-SO₂)(Rf²-SO₂)NH aqueux tel qu'obtenu selon le procédé décrit ci-dessus, en présence d'une base de métal alcalin, et notamment une base lithiée, le cas échéant suivi d'une étape de séchage.

La présente invention a également pour objet un procédé de préparation de (Rf¹SO₂)(Rf²SO₂)NM^{b}, de préférence de (CF₃SO₂)₂NLi, comprenant les étapes suivantes :
- une étape a) de salification de Rf¹CO₂H en Rf¹CO₂M^{a} ;
- une étape b) de sulfination de Rf¹CO₂M^{a} en Rf¹SO₂M^{a} ;
- une étape c) d'oxydation de Rf¹SO₂M^{a} en Rf¹SO₂X ;
- une étape d) d'ammonolyse de Rf¹SO₂X en (Rf¹SO₂)(Rf²-SO₂)NH,NR'₃;
- une étape e) d'acidification de (Rf¹SO₂)(Rf²SO₂)NH,NR'₃ en (Rf¹SO₂)(Rf²SO₂)NH;
- une étape g) de neutralisation, par une base de métal alcalin, de (Rf¹SO₂)(Rf²SO₂)NH en (Rf¹SO₂)(Rf²-SO₂)NM^{b}; et
- le cas échéant une étape h) de séchage de (Rf¹SO₂)(Rf²SO₂)NM^{b},
dans lequel (Rf¹-SO₂)(Rf²-SO₂)NH, obtenu à l'issue de l'étape e), est sous forme d'un mélange M1 comprenant (Rf¹SO₂)(Rf²SO₂)NH et Rf¹SO₂H et/ou Rf²SO₂H, et (Rf¹SO₂₎(Rf²SO₂)NH est purifié entre l'étape e) et l'étape g) par la mise en oeuvre du procédé tel que définit précédemment; Rf¹, Rf², M^{a} et M^{b} étant tels que définis ci-dessus.

Selon un mode de réalisation particulier, la présente invention concerne un procédé de préparation de (CF₃SO₂)₂NLi, comprenant les étapes suivantes :
- une étape a) de salification de CF₃CO₂H en CF₃CO₂K;
- une étape b) de sulfination de CF₃CO₂K en CF₃SO₂K;
- une étape c) d'oxydation de CF₃SO₂K en CF₃SO₂X ;
- une étape d) d'ammonolyse de CF₃SO₂X en (CF₃SO₂)₂NH,NR'₃;
- une étape e) d'acidification de (CF₃SO₂)₂NH,NR'₃ en (CF₃SO₂)₂NH;
- une étape g) de neutralisation, par une base lithiée, de (CF₃SO₂)₂NH en (CF₃SO₂)₂NHLi; et
- le cas échéant une étape h) de séchage de (CF₃SO₂)₂NHLi,
dans lequel (CF₃SO₂)₂NH, obtenu à l'issue de l'étape e), est sous forme d'un mélange comprenant (CF₃SO₂)₂NH et CF₃SO₂H, ledit mélange ayant été soumis, avant l'étape g), à une étape d'oxydation i) et de distillation ii) telles que décrites précédemment.

La présente invention a également pour objet un procédé de préparation de (Rf¹-SO₂)(Rf²-SO₂)NM^{b} comprenant les étapes suivantes :
- une étape b') de transformation de R^{h}SO₂W, avec W représentant Cl, Br ou I, en R^{h}SO₂F ;
- une étape c') d'électrofluoration de R^{h}SO₂F en Rf¹SO₂F ;
- une étape d) d'ammonolyse de Rf¹SO₂F en (Rf¹SO₂)(Rf²-SO₂)NH,NR¹₃;
- une étape e) d'acidification de (Rf¹SO₂)(Rf²SO₂)NH,NR'₃ en (Rf¹SO₂)(Rf²SO₂)NH;
- une étape g) de neutralisation, par une base de métal alcalin, de (Rf¹SO₂)(Rf²SO₂)NH en (Rf¹-SO₂)(Rf²-SO₂)NM^{b}; et
- le cas échéant une étape h) de séchage de (Rf¹SO₂)(Rf²SO₂)NM^{b},
dans lequel (Rf¹-SO₂)(Rf²-SO₂)NH, obtenu à l'issue de l'étape e), est sous forme d'un mélange M1 comprenant (Rf¹SO2)(Rf²SO₂)NH et Rf¹SO₂H et/ou Rf²SO₂H, et (Rf¹SO₂)(Rf²SO₂)NH est purifié entre l'étape e) et l'étape g) par la mise en oeuvre du procédé tel que décrit précédemment; et Rf¹, R^{h}, Rf², M^{b} étant tels que définis ci-dessus.

Selon l'invention, les différentes étapes du procédé mentionnées ci-dessus, et détaillés ci-après, et notamment les étapes de sulfination, d'oxydation et d'ammonolyse, peuvent être réalisées dans un réacteur continu ou discontinu.

Selon l'invention, les différentes réactions peuvent être réalisées dans un réacteur à écoulement piston, et par exemple un réacteur tubulaire unique, ou une cascade de réacteurs parfaitement agités.

Dans le cadre de l'invention, et sauf mention contraire, on entend par « réacteur tubulaire », un réacteur en forme de tube.

Dans le cadre de l'invention, et sauf mention contraire, on entend par « réacteur à écoulement piston », un réacteur dans lequel l'écoulement est unidirectionnel, et dans lequel dans un plan perpendiculaire à l'écoulement, tous les filets fluides se déplacent avec une vitesse uniforme. Dans un tel écoulement, le mélange radial est parfait alors qu'il n'y a pas de mélange axial.

Les différentes étapes sont détaillées ci-dessous.

### Etape de salification a)

Dans le cadre de l'invention, le procédé de préparation de (Rf¹SO₂)(Rf²SO₂)NM^{b} peut comprendre une étape a) de salification de Rf¹CO₂H en Rf¹CO₂M^{a}.

L'étape de salification de Rf¹CO₂H en Rf¹CO₂M^{a} peut être réalisée selon les procédés connus de l'homme du métier.

Selon un mode de réalisation, M^{a} est choisi parmi le groupe constitué de : K, Li, Na, Cs. De préférence, M^{a} représente K.

Selon un mode de réalisation, Rf¹CO₂H est CF₃CO₂H,

Selon un mode de réalisation, Rf¹CO₂K est CF₃CO₂K.

Dans le cadre de l'invention, et sauf mention contraire, on entend par «salification», la réaction visant à préparer le sel de métal alcalin Rf¹CO₂M^{a}, à partir de l'acide Rf¹CO₂H. On peut par exemple citer la salification de l'acide trifluoroacétique CF₃CO₂H en trifluoroacétate de potassium CF₃CO₂K ou la salification de C₂F₅CO₂H en C₂F₅CO₂K

Selon un mode de réalisation, l'étape de salification a) de Rf¹CO₂H en Rf¹CO₂M^{a}, consiste en l'addition lente de Rf¹CO₂H dans une solution alcaline aqueuse, et notamment une solution d'hydroxyde de potassium aqueuse. L'ajout est notamment réalisé pendant une durée suffisante pour que la température du milieu réactionnel reste comprise entre 30°C et 80°C.

A l'issue de l'étape de salification a), une solution de Rf¹CO₂M^{a}, de préférence Rf¹CO₂K et préférentiellement de trifluoroacétate de potassium, dans l'eau peut être obtenue.

Selon un mode de réalisation, une étape de concentration de la solution de Rf¹CO₂M^{a} est mise en oeuvre en fin de l'étape de salification a), de façon à éliminer l'eau formée au cours de la réaction. De plus, l'étape de distillation peut être également suivie d'une étape de séchage, notamment dans une étuve à une température comprise entre 30°C et 100°C, préférentiellement sous vide.

De préférence, la solution de Rf¹CO₂M^{a} obtenue après concentration comprend une quantité d'eau inférieure à 10000 ppm, de préférence inférieure à 1000 ppm et préférentiellement inférieure à 500 ppm.

### Etape de sulfination b)

Dans le cadre de l'invention, le procédé de préparation de (Rf¹SO₂)(Rf²SO₂)NM^{b}, et notamment de (Rf¹SO₂(Rf²SO₂)₂NLi, peut comprendre une étape b) de sulfination de Rf¹CO₂M^{a} en Rf¹SO₂M^{a}.

Dans le cadre de l'invention, et sauf mention contraire, on entend par « sulfination », la réaction visant à préparer le Rf¹SO₂M^{a} à partir de Rf¹CO₂M^{a}. On peut par exemple citer la réaction de sulfination du trifluoroacétate de potassium (CF₃CO₂K) en triflinate de potassium (CF₃SO₂K) ou la réaction de sulfination de C₂F₅SO₂K à partir de C₂F₅CO₂K.

Selon un mode de réalisation, M^{a} représente K. Ainsi, de préférence Rf¹SO₂M^{a} est Rf¹SO₂K.

L'étape de sulfination de Rf¹CO₂M^{a} en Rf¹SO₂M^{a} peut être réalisée selon les procédés connus de l'homme du métier. La réaction b) de sulfination selon l'invention peut notamment être réalisée dans les conditions décrites dans FR 2900403, FR2732010 ou FR2732016.

Selon un mode de réalisation, l'étape b) de sulfination du procédé consiste à faire réagir le Rf¹CO₂M^{a} avec un oxyde de soufre.

Selon l'invention, l'oxyde de soufre, de préférence le dioxyde de soufre, peut être mis en oeuvre sous forme gazeuse. Il peut être également introduit sous forme de solution, dans le solvant organique choisi pour la réaction (solvant réactionnel), à une concentration variant généralement entre 1 et 10 % en poids, de préférence entre 3 et 6 %.

De préférence, la réaction de sulfination est réalisée en présence de SO₂ sous forme gazeuse.

Selon un mode de réalisation, le rapport entre le nombre de moles d'oxyde de soufre et le nombre de moles de Rf¹CO₂M^{a}, et notamment de CF₃CO₂K va de 0,1 à 5, de préférence de 1 à 3. Préférentiellement, le rapport est d'environ 2.

L'étape b) de sulfination du procédé de l'invention peut être réalisée dans un solvant organique aprotique. De préférence, le solvant utilisé dans l'étape de sulfination est un solvant aprotique polaire. De préférence, le solvant aprotique polaire comporte très peu d'impuretés porteuses d'hydrogène acide.

Dans le cadre de l'invention, et sauf mention contraire, on entend par « solvant aprotique », un solvant qui, dans la théorie de Lewis, n'a pas de protons à libérer.

Selon un mode de réalisation, le solvant utilisé permet de solubiliser le Rf¹CO₂M^{a} au moins partiellement, de préférence complètement. De préférence, le solvant aprotique polaire utilisable a un moment dipolaire significatif. Ainsi, sa constante diélectrique relative ε est avantageusement au moins égale à environ 5. De préférence, sa constante diélectrique est inférieure ou égale à 50 et supérieure ou égale à 5, et est notamment comprise entre 30 et 40.

Afin de déterminer si le solvant organique répond aux conditions de constante diélectrique énoncées ci-dessus, il est possible de se reporter, entre autres, aux tables de l'ouvrage : Techniques of Chemistry, II - Organic solvents - p. 536 et suivantes, 3ème édition (1970).

Selon un mode de réalisation, le solvant organique de l'étape de sulfination est susceptible de bien solvater les cations, ce qui signifie que le solvant présente certaines propriétés de basicité au sens de Lewis. Afin de déterminer si le solvant satisfait à cette exigence, sa basicité est appréciée en se référant au "nombre donneur". En particulier, le solvant organique polaire utilisé présente un nombre donneur supérieur à 10, de préférence supérieur ou égal à 20. De préférence, le solvant organique a un nombre donneur compris entre 10 et 30.

Dans le cadre de l'invention, et sauf mention contraire, on entend par "nombre donneur" ou "donor number" désigné de manière abrégée DN, un nombre qui donne une indication sur le caractère nucléophile du solvant et révèle son aptitude à donner son doublet.

Dans l'ouvrage de Christian REICHARDT, [Solvents and Solvent Effects in Organic Chemistry - VCH p.19 (1990)], la définition du "donor number" est définie comme le négatif (-ΔH) de l'enthalpie (Kcal/mol) de l'interaction entre le solvant et le pentachlorure d'antimoine, dans une solution diluée de dichloroéthane.

Dans l'étape b) de sulfination selon la présente invention, le ou les solvants polaires peuvent ne pas présenter d'hydrogène acide, en particulier lorsque le caractère polaire du ou des solvants est obtenu par la présence de groupes électro-attracteurs, il est souhaitable qu'il n'y ait pas d'hydrogène sur l'atome en position α de la fonction électro-attractrice.

De manière plus générale, il est préférable que le pKa correspondant à la première acidité du solvant soit au moins égal à environ 20 ("environ" soulignant que seul le premier chiffre des dizaines est significatif), avantageusement compris entre 20 et 35, et de préférence compris entre 25 et 35.

Le caractère acide peut également être exprimé par l'indice accepteur AN du solvant, tel qu'il est défini par Christian REICHARDT, [Solvents and solvant affects in Organic Chemistry", 2ème édition, VCH (RFA), 1990, pages 23-24].

Avantageusement, cet indice accepteur AN est inférieur à 20, en particulier inférieur à 18.

Les solvants répondant aux différents impératifs et donnant de bons résultats, dans l'étape b) de sulfination, peuvent être notamment des solvants de type amide. Parmi les amides, on comprend aussi les amides à caractère particulier comme les urées tétrasubstituées et les lactames monosubstitués. Les amides sont de préférence substitués (disubstitués pour les amides ordinaires). On peut citer par exemple les dérivés de pyrrolidone, tels que la N-méthylpyrrolidone (NMP), ou encore le N,N-diméthylformamide (DMF) ou le N,N-diméthylacétamide.

Une autre catégorie particulièrement intéressante de solvant est constituée par les éthers, qu'ils soient symétriques ou non symétriques, qu'ils soient ouverts ou non. Dans la catégorie des éthers doivent être incorporés les différents dérivés des éthers de glycol tels que les différents glymes, le diglyme par exemple.

Préférentiellement, le DMF est le solvant utilisé dans l'étape b) de sulfination.

Selon l'Invention, la mise en oeuvre peut être effectuée de manière discontinue (en batch) ou d'une manière continue.

Selon un mode de mise en oeuvre en discontinu, Rf¹CO₂M^{a} est introduit dans le solvant organique puis l'oxyde de soufre est ajouté en totalité ou par fractions.

Selon l'invention, la réaction de sulfination peut être conduite dans un réacteur classique équipé d'un dispositif de chauffage (échangeur thermique) et d'un dispositif d'agitation, par exemple, une agitation à l'aide d'une hélice. Ensuite, le mélange réactionnel est chauffé.

Selon un mode de réalisation en continu, on fait appel à un appareillage permettant une mise en oeuvre en continu tel que plusieurs réacteurs en cascade ou un tube équipé d'une double enveloppe dans lequel circule un fluide caloporteur dont les caractéristiques permettent d'atteindre la température réactionnelle souhaitée.

Dans ce cas, le dispositif peut être alimenté par Rf¹CO₂M^{a} en mélange avec le solvant organique et le dioxyde de soufre peut alors être introduit. Ce dernier peut être ajouté dans la solution d'alimentation comprenant le Rf¹CO₂M^{a} et le solvant organique ou bien il est peut être introduit à différents endroits de l'appareillage : l'arrivée pouvant être faite dans le ciel du réacteur ou dans la masse réactionnelle.

Ensuite, le chauffage peut être effectué jusqu'à obtention du taux de transformation souhaité.

Selon un mode de réalisation, l'étape b) de sulfination peut être effectuée à une température allant de 100°C à 200°C, de préférence de 110°C à 180°C, préférentiellement de 120°C à 150°C. De préférence, la réaction de sulfination est réalisée à 140°C.

La réaction de sulfination est avantageusement mise en oeuvre sous pression atmosphérique mais des pressions plus élevées peuvent être également utilisées. Ainsi, la réaction peut être réalisée à une pression totale absolue choisie entre 1 et 20 Bar (100 à 2000 kPa) et de préférence entre 1 et3 Bar (100 à 300kPa).

La durée du chauffage peut varier largement en fonction de la température de réaction choisie. En particulier, la réaction est effectuée pendant environ 30 min à 24 heures, de préférence pendant deux heures à moins de 20 heures, et plus préférentiellement pendant 4 heures à 5 heures.

Lorsque ledit oxyde de soufre est du dioxyde de soufre, le mélange résultant de l'étape b) de sulfination peut comprendre deux phases : une phase liquide, où une partie au moins dudit Rf¹CO₂M^{a} et du dioxyde de soufre sont dissous dans ledit solvant et une phase gazeuse contenant essentiellement du dioxyde de soufre et du gaz carbonique formé au cours de la réaction.

Pour éviter une dégradation trop importante du produit final, et donc assurer une bonne sélectivité de la réaction, il est préférable de ne pas chercher à convertir complètement Rf¹CO₂M^{a} de départ. L'avancement de la réaction peut être contrôlé par le taux de conversion (TT) du Rf¹CO₂M^{a} qui est le rapport molaire de la quantité de Rf¹CO₂M^{a} disparue sur la quantité de Rf¹CO₂M^{a} initiale dans le milieu réactionnel, ce taux étant calculé aisément après dosage de l'acide restant dans le milieu.

De préférence, la réaction de sulfination est conduite jusqu'à l'obtention d'un taux de transformation supérieur à 30% exprimée par le rapport molaire produit recherché Rf¹SO₂M^{a} / Rf¹CO₂M^{a} transformé.

Dans le cadre de l'invention, et sauf mention contraire, on entend par « taux de conversion (TT) », le rapport entre le nombre de moles de substrat transformées et le nombre de moles de substrat engagées.

Dans le cadre l'invention, et sauf mention contraire, on entend par « rendement (RR) », le rapport entre le nombre de moles de produit formées et le nombre de moles de substrat engagées.

Dans le cadre l'invention, et sauf mention contraire, on entend par « sélectivité (RT) », le rapport entre le nombre de moles de produit formées et le nombre de moles de substrat transformées au cours de la réaction.

Dans le cadre de l'invention, le procédé peut comprendre une étape de détente après l'étape b) de sulfination.

Selon un mode de réalisation, l'étape b) de sulfination conduit à un mélange M consistant notamment en une solution de Rf¹CO₂M^{a} non transformé et de Rf¹SO₂M^{a} formé, dans le solvant de la réaction, notamment dans le DMF.

Selon un mode de réalisation, le mélange M obtenu à l'issue de l'étape b) est soumis à une étape de distillation du solvant réactionnel, notamment du DMF. De manière avantageuse, le solvant réactionnel distillé peut être recyclé et réutilisé dans l'étape b) de sulfination.

### Etape de séparation des sels et recyclage du solvant organique b1)

Dans le cadre de l'invention, le procédé selon l'invention peut comprendre à l'issue de l'étape b) de sulfination, une étape b1) de séparation des sels et recyclage du solvant organique, par exemple par extraction liquide/liquide.

L'étape d'extraction b1) peut être réalisée selon les procédés connus de l'homme du métier.

Selon un mode de réalisation, l'étape b1) est réalisée par ajout d'eau et d'un solvant d'extraction sur le milieu réactionnel résultant de l'étape précédente.

De préférence, le solvant d'extraction est un solvant chloré, notamment choisi parmi le dichiorométhane, le chloroforme, le tétrachlorure de carbone, le 1,2-dichloroéthane, le trichloroéthylène et le perchloroéthylène, ainsi que les solvants aromatiques chlorés (chlorobenzène, 1,2-dichlorobenzène et homologues supérieurs). De préférence, le solvant d'extraction est le dichlorométhane.

Dans le cadre de l'invention, un milieu biphasique peut être obtenu contenant une phase organique O3 et une phase aqueuse saline A3, après décantation desdites phases.

Selon un mode de réalisation, la phase O3 comprend le solvant d'extraction, notamment le dichlorométhane, ainsi que le solvant organique utilisé dans l'étape b) de sulfination, notamment le DMF.

Dans le cadre de l'invention, les phases O3 et A3 peuvent être séparées après décantation, par l'étape b1) d'extraction liquide/liquide desdites phases.

De préférence, la phase O3 a une densité inférieure à celle de la phase aqueuse A3.

Dans le cadre de l'invention, la phase O3 peut être soumise à une étape de distillation permettant de récupérer séparément le solvant d'extraction, notamment le dichlorométhane, et le solvant organique utilisé dans l'étape b) de sulfination, notamment le DMF. Les différents solvants peuvent être recueillis séparément en raison de leurs points d'ébullition différents.

Selon un mode de réalisation, la phase aqueuse A3 comprend un mélange M3 comprenant Rf¹CO₂M^{a} et Rf¹SO₂M^{a}.

Le procédé selon l'invention peut comprendre une étape de recyclage du solvant utilisé dans l'étape b) de sulfination, notamment du DMF, obtenu par distillation de la phase O3. Le solvant ainsi recyclé peut être réinjecté dans le procédé, notamment dans l'étape b) de sulfination.

Le procédé selon l'invention peut également comprendre une étape de recyclage du solvant d'extraction, notamment du dichlorométhane, obtenu par distillation de la phase O3. Le solvant d'extraction ainsi recyclé peut être réinjecté dans le procédé, à savoir dans l'étape b1)..

Dans le cadre de l'invention, et sauf mention contraire, on entend par « solvant recyclé », un solvant qui a subi une étape de recyclage.

Cette étape b1) peut permettre avantageusement de récupérer les sels Rf¹CO₂M^{a} (non transformé dans l'étape de sulfination) et Rf¹SO₂M^{a} (formé), dans une phase aqueuse, notamment dans la phase aqueuse A3.

De plus, l'utilisation d'un solvant d'extraction permet avantageusement d'éliminer le solvant organique utilisé dans l'étape b) de sulfination.

Le procédé de préparation de (Rf¹SO₂)(Rf²SO₂)NM^{b}, et notamment de (CF₃SO₂)₂NLi selon l'invention, comprend avantageusement des étapes de recyclage de solvants, tels que le solvant d'extraction de l'étape d'extraction b1) ou le solvant réactionnel de l'étape b) de sulfination.

### Etape c) d'oxydation

Le procédé de préparation de (Rf¹SO₂)(Rf²SO₂)NM^{b} selon l'invention peut comprendre une étape c) d'oxydation de la phase aqueuse A3 telle que définie ci-dessus, pour conduire à Rf¹SO₂X, X représentant un atome de chlore, de fluor, ou de brome.

Selon un mode de réalisation, Rf¹SO₂X est CF₃SO₂X, X étant tel que défini ci-dessus.

En particulier, l'étape d'oxydation de la phase aqueuse A3, correspond à l'oxydation du mélange M3 comprenant Rf¹CO₂M^{a} et Rf¹SO₂M^{a}.

L'étape d'oxydation peut être réalisée selon les procédés connus de l'homme du métier.

Selon un mode de réalisation, l'étape d'oxydation de la phase aqueuse A3 est réalisée en présence d'un agent oxydant choisi parmi SO₂F₂, F₂, Cl₂, SO₂Cl₂, Br₂ et SO₂Br₂

Selon un mode de réalisation, lorsque l'agent oxydant est SO₂Cl₂ ou Cl₂, le produit formé est Rf¹SO₂Cl, et notamment le chlorure de trifluorométhanesulfonyle CF₂SO₂Cl.

Selon un autre mode de réalisation, lorsque l'agent oxydant est SO₂Br₂ ou Br₂, le produit formé est Rf¹SO₂Br, et notamment le bromure de trifluorométhanesulfonyle CF₃SO₂Br.

Selon un autre mode de réalisation, lorsque l'agent oxydant est SO₂F₂ ou F₂, le produit formé est le Rf¹SO₂F, et notamment le fluorure de trifluorométhanesulfonyle CF₃SO₂F.

De préférence, l'agent oxydant est Cl₂ et le produit formé au cours de l'étape c) d'oxydation est Rf¹SO₂Cl.

Selon l'invention, l'étape d'oxydation peut être réalisée à une température allant de -40°C à 20°C, de préférence de -20°C à 5°C, et préférentiellement de -5°C à 0°C.

Selon un mode de réalisation, l'étape d'oxydation de la phase aqueuse A3, conduit à un mélange M4 biphasique comprenant une phase aqueuse A4.

Selon un mode de réalisation particulier, lorsque l'agent oxydant est choisi parmi SO₂F₂ ou F₂, l'étape d'oxydation de la phase aqueuse A3, conduit à un mélange M4 comprenant une phase aqueuse A4 et une phase gazeuse G4. En particulier, la phase gazeuse G4 comprend, préférentiellement est constituée de, Rf¹SO₂F.

Selon l'invention, Rf¹SO₂F gazeux peut être condensé, notamment à une température d'environ -80°C à -30°C. Typiquement, Rf¹SO₂F condensé peut être purifié par cryodistillation ou traité par de l'acide sulfurique.

Selon un autre mode de réalisation particulier, lorsque l'agent oxydant est choisi parmi Cl₂, SO₂Cl₂, Br₂ et SO₂Br₂, l'étape d'oxydation de la phase aqueuse A3, conduit à un mélange M4 comprenant après décantation, une phase organique O4 et une phase aqueuse A4. En particulier, la phase organique a une densité inférieure à celle de la phase aqueuse A4.

De préférence, la phase organique O4 comprend Rf¹SO₂X, notamment Rf¹SO₂Cl ou Rf¹SO₂Br, formé à l'issue de l'étape c) d'oxydation, qui décante sous la forme d'un liquide immiscible dans l'eau. La phase O4 peut être soumise à une étape de distillation, de manière à récupérer Rf¹SO₂X, et notamment Rf¹SO₂Cl.

De préférence, ladite phase aqueuse A4 comprend Rf¹CO₂M^{a} et des sels, notamment des sels d'halogénures. En particulier, les sels d'halogénures sont choisis parmi le chlorure de potassium, le fluorure de potassium ou le bromure de potassium. La nature de ces sels d'halogénures dépend notamment de la nature de l'agent d'oxydation utilisé. Le Rf¹CO₂M^{a} de la phase A4 correspond au Rf¹CO₂M^{a} de la phase aqueuse A3, et plus particulièrement du mélange M3, qui n'a pas été transformé lors de l'étape c) d'oxydation.

Selon le procédé de l'invention, la phase A4 peut contenir de 1% à 45% en poids de Rf¹CO₂M^{a}, de préférence de 5% à 30% et préférentiellement de 10% à 25%.

Typiquement, la phase organique O4 peut être séparée de la phase A4 par décantation.

Selon l'invention, le composé Rf¹SO₂F peut être obtenu par oxydation d'un mélange M3, comprenant Rf¹CO₂M^{a} et Rf¹SO₂M^{a}, tel que défini ci-dessus, ou par électrofluoration de R^{h}SO₂F, R^{h} représentant une chaîne hydrocarbonée identique à celle de Rf¹, ladite chaîne comprenant des atomes d'hydrogène à la place des atomes de fluor, R^{h}SO₂F étant éventuellement obtenu à partir de R^{h}SO₂W, avec W représentant un atome d'halogène différent du fluor, à savoir Br, Cl ou I, et de préférence le chlore.

Selon l'invention, le composé Rf¹SO₂F peut également être obtenu à partir de Rf¹SO₂Cl. Rf¹SO₂Cl peut notamment être obtenu selon les procédés décrits dans WO 2008/111418, WO 2009/060815 et Jpn. Kokai Tokkyo Koho, 2010173959, 12/08/2010.

Typiquement, l'étape d'électrofluoration de R^{h}SO₂F peut être réalisée dans les conditions connues de l'homme du métier, et notamment dans les conditions décrites dans US 4,927,962.

Typiquement, le composé R^{h}SO₂F peut être préparé dans les conditions connues de l'homme du métier, et notamment par échange d'halogène à partir de R^{h}SO₂W, W représentant un atome d'halogène différent du fluor, de préférence le chlore, les conditions d'échange étant notamment décrites dans US 5,540,818.

En particulier, R^{h}SO₂F est CH₃SO₂F.

En particulier, R^{h}SO₂W est CH₃SO₂Cl.

Selon un mode de réalisation, CF₃SO₂F est obtenu par électrofluoration de CH₃SO₂F, CH₃SO₂F étant obtenu par réaction d'échange d'halogène de CH₃SO₂Cl en milieu aqueux fluoré, tel que décrit dans US 5,540,818.

Selon l'invention, le procédé selon l'invention, peut comprendre le recyclage de Rf¹CO₂M^{a}. En effet, Rf¹CO₂M^{a} qui n'a pas été transformé au cours de l'étape c) d'oxydation de la phase aqueuse A3, peut étre recyclé et réutilisé dans le procédé.

Il a été avantageusement montré que l'utilisation d'un agent oxydant tel que Cl₂ ou F₂, permet d'agir sélectivement sur le composé Rf¹SO₂M^{a} d'un mélange contenant Rf¹SO₂M^{a} et Rf¹CO₂M^{a}, sans agir sur Rf¹CO₂M^{a}. Ainsi, le Rf¹CO₂M^{a} peut être avantageusement recyclé. Ceci permet de valoriser le Rf¹CO₂M^{a} au sein du procédé et permet une économie globale du procédé.

### Etape de recyclage de Rf¹CO₂M^{a}

Typiquement, l'étape de recyclage de Rf¹CO₂M^{a} peut être réalisée dans les conditions connues de l'homme du métier, et notamment dans les conditions décrites dans FR 2 924 116.

Selon un mode de réalisation, le recyclage de Rf¹CO₂M^{a} comprend différentes étapes, et notamment une étape d'acidification de la solution aqueuse A4 dans un éther, pour obtenir un mélange biphasique M5.

Selon l'invention, l'éther peut être choisi parmi : l'anisole, le diisopropyléther (DIPE), le méthyl-terbutyléther (MTBE), le diphényléther et les alkyle-éthers, tels que le n-butyléther. De préférence, l'éther est le MTBE.

Selon l'invention, l'étape d'acidification peut être réalisée avec un acide choisi parmi l'acide chlorhydrique, l'acide sulfurique, l'acide nitrique ou l'acide phosphorique. De préférence, l'acide est l'acide chlorhydrique.

Selon un mode de réalisation, l'acide est ajouté de façon à acidifier la solution aqueuse A4 contenant Rf¹CO₂M^{a} jusqu'à un pH compris de 0 à 5, de 1 à 3, et préférentiellement jusqu'à un pH égal à 1.

Selon un mode de réalisation, l'éther est ajouté à la solution aqueuse acidifiée.

Selon le procédé de l'invention, le mélange biphasique M5 obtenu à l'issue de l'étape d'acidification, peut comprendre une phase organique O5 comprenant l'éther, et une phase aqueuse A5 comprenant des sels, notamment des sels d'halogénure.

De préférence, la phase organique O5 comprend également Rf¹CO₂H formé pendant l'étape d'acidification. En particulier, ta phase O5 comprend un complexe formé entre l'éther et Rf¹CO₂H.

Selon un mode de réalisation, la phase O5 comprend de 10% à 70% en poids de Rf¹CO₂H, et de préférence de 30% à 60% poids.

De préférence, la phase A5 comprend des sels, notamment des sels d'halogénure, tels que KF, KBr ou KCl. La nature de ces sels dépend notamment de l'agent oxydant utilisé dans l'étape d'oxydation susmentionnée.

Selon l'invention, les phases O5 et A5 peuvent être séparées par décantation. De préférence, la phase O5 a une densité inférieure à celle de la phase A5.

Il a été montré qu'il se forme un complexe entre l'éther et le Rf¹CO₂H formé, qui décante et permet de déplacer l'équilibre vers la formation de Rf¹CO₂H.

Cette étape d'acidification permet avantageusement de purifier Rf¹CO₂H, en le séparant des sels d'halogénures restés dans la phase aqueuse A5.

L'étape de recyclage de Rf¹CO₂M^{a}, peut comprendre en outre une étape de transformation de Rf¹CO₂H, formé lors de l'étape d'acidification, en Rf¹CO₂M^{a}. Typiquement, cette étape de transformation de Rf¹CO₂H en Rf¹CO₂M^{a} est une étape de basificatton.

Selon un mode de réalisation, une solution aqueuse d'hydroxyde de métal alcalin, notamment KOH, est ajoutée à la phase organique O5 précédente.

Selon l'invention, une solution aqueuse d'hydroxyde de métal alcalin, notamment KOH aqueux, peut être ajoutée à la phase O5 jusqu'à obtenir un pH final supérieur à 7,5.

A l'issue de l'étape de basification de la phase O5, un mélange biphasique M6 peut être obtenu.

De préférence, le mélange M6 comprend une phase organique O6 et une phase aqueuse A6, la phase organique O6 comprenant l'éther utilisé dans l'étape d'acidification précédente, et la phase A6 comprenant Rf¹CO₂M^{a} formé et l'hydroxyde de métal alcalin, notamment KOH, en excès. Typiquement, ces deux phases sont séparées par décantation.

Selon l'invention, la phase O6 peut être recyclée. De préférence, la phase O6, comprenant l'éther, est recyclée pour être réutilisée dans l'étape d'acidification telle que définie ci-dessus.

En particulier, la phase A6 comprend de 5% à 55% en poids de Rf¹SO₂M^{a}, de préférence de 5% à 45% poids, et préférentiellement de 10% à 40% poids.

Dans le cadre de l'invention, l'étape de recyclage de Rf¹CO₂M^{a} peut comprendre une étape supplémentaire de neutralisation de la phase A6. En particulier, cette étape de neutralisation consiste en l'ajout de Rf¹CO₂H dans la phase A6, pour conduire à une phase aqueuse A7 ayant un pH neutre.

Selon un mode de réalisation, la quantité de Rf¹CO₂H ajoutée dans la phase A6 est égale à la quantité d'hydroxyde de métal alcalin, notamment KOH, présente dans ladite phase A6.

Dans le cadre de l'invention, et sauf mention contraire, la phase A7 correspond à la phase A6 qui a subi une étape de neutralisation. La phase A7 peut être obtenue par ajout de R¹CO₂H dans la phase A6.

Selon un mode de réalisation, la phase A7 comprend Rf¹CO₂M^{a}. En particulier, la phase A7 comprend de 5% à 50% en poids de Rf¹CO₂M^{a}, de préférence de 8% à 45% poids, et préférentiellement de 10% à 40% poids.

Dans le cadre de l'invention, l'étape de recyclage de Rf¹CO₂M^{a} peut comprendre une étape visant à séparer Rf¹CO₂M^{a} de la phase aqueuse A7.

Ainsi, le procédé peut comprendre une étape d'évaporation de l'eau de la phase A7.

De préférence, un précipité de Rf¹CO₂M^{a} est obtenu, ledit précipité comprenant jusqu'à 10% en poids d'eau, de préférence jusqu'à 7% en poids.

Selon un mode de réalisation, l'étape de recyclage de Rf¹CO₂M^{a} peut comprendre une étape d'ajout d'un solvant organique, de préférence d'un solvant organique tel que défini précédemment pour l'étape de sulfination, tel que le DMF ou la NMP. En particulier, le solvant organique est le DMF. Typiquement, l'ajout de DMF permet de faciliter l'évaporation d'eau restante dans le précipité.

En particulier, l'ajout de DMF sur le précipité de Rf¹CO₂M^{a} permet de conduire à un mélange M8, comprenant notamment de l'eau.

Selon un mode de réalisation, une étape d'évaporation de l'eau du mélange M8 est réalisée.

Dans le cadre de l'invention, une solution O8 peut être obtenue à l'issue de l'étape d'évaporation de l'eau du mélange M8.

De préférence, la solution O8 est une solution de Rf¹CO₂M^{a} dans le solvant organique, notamment dans le DMF. En particulier, la solution O8 comprend de 10% à 60% en poids de Rf¹CO₂M^{a} et de préférence de 20% à 50% poids.

Selon l'invention, la solution O8 peut comprendre une quantité d'eau inférieure à 500 ppm, préférentiellement inférieure à 300 ppm. En particulier, la solution O8 comprend une quantité d'eau inférieure à 200 ppm.

Dans le cadre de l'invention, la solution O8 de Rf¹CO₂M^{a} dans le solvant organique, notamment le DMF, peut être recyclée dans le procédé de préparation de (Rf¹SO₂)(Rf²SO₂)NM^{b}, et notamment de (CF₃SO₂)₂NLi. En particulier, la solution O8 peut être réutilisée dans l'étape b) de sulfination.

### Etape d) d'ammonolyse

Dans le cadre de l'invention, le procédé de préparation de (Rf¹SO₂)(Rf²SO₂)NM^{b} peut comprendre une étape d) d'ammonolyse de Rf¹SO₂X tel que défini précédemment

L'étape d'ammonolyse de Rf¹SO₂X peut être réalisée selon les procédés connus de l'homme du métier.

De préférence, Rf¹SO₂X est tel que X représente un atome d'halogène, notamment Br, Cl ou F. En particulier Rf¹SO₂X est Rf¹SO₂Cl ou Rf¹SO₂F, et préférentiellement Rf¹SO₂X est Rf¹SO₂Cl.

La réaction d'ammonolyse peut notamment être réalisée dans les conditions décrites dans FR 2763331 ou dans FR 2724380.

Selon un mode de réalisation, l'étape d'ammonolyse est réalisée selon deux modes opératoires différents, tels que ceux décrits dans FR2724380, chacun des deux modes pouvant conduire à des sulfonimides symétriques ou dissymétriques.

Selon l'invention, l'étape d'ammonolyse peut être réalisée :
iii) soit en présence de Rf¹SO₂X et Rf²SO₂X, de NH₃ et d'une base choisie parmi les amines tertiaires, les trialcoylphosphines, les dialcoylphosphines encombrées, les hydroxydes de phosphonium, les dialcoylamines encombrées, trialcoylamines, les hydroxydes d'ammonium, les cycles phosphorés et azotés présentant une basicité adéquate ;
iv) soit en présence de Rf¹SO₂X, d'un sulfonamide de formule Rf²SO₂NH₂ Rf¹ et Rf² pouvant être identiques ou différents, et d'une base telle que définie ci-dessus selon la méthode iii).

Selon l'invention, l'étape d'ammonolyse peut conduire à un triflimidure de formule suivante : [(Rf¹SO₂)₂N⁻, R'₃NH⁺] ou [(Rf¹SO₂)(Rf²SO₂)N⁻, R'₃NH⁺], dans laquelle Rf₁ et Rf₂ peuvent être identiques ou différents.

Le respect de la stoechiométrie de la réaction d'ammonolyse est souhaitable en ce qui concerne Rf¹SO₂X et la base utilisée. La réaction d'ammonolyse peut néanmoins être mise en oeuvre avec succès en utilisant un excès de base.

Selon l'invention, la réaction d'ammonolyse selon la méthode iii) peut être réalisée en présence d'au moins trois équivalents de base, et :
- soit d'au moins deux équivalents de Rf¹SO₂X, notamment Rf¹SO₂Cl,
- soit d'au moins un équivalent de Rf¹SO₂X et d'au moins un équivalent de Rf¹SO₂X, Rf¹ et Rf² étant identiques ou différents.

Selon l'invention, Rf²SO₂X peut être obtenu selon les mêmes conditions que celles mises en oeuvre pour préparer Rf¹SO₂X telles que décrites ci-dessus

Selon l'invention, Rf²SO₂NH₂ peut être obtenu selon les procédés connus de l'homme du métier, et notamment selon les conditions décrites par Foropoulos et al. (Inorganic Chemistry, 1984, vol. 23, N° 23, p. 3720-3723). En particulier, Rf²SO₂NH₂ est avantageusement préparée par réaction de Rf²SO₂X (X = halogène, notamment F, Cl et Br) avec de l'ammoniac à -78°C.

Ainsi, l'emploi d'au moins deux équivalents de Rf¹SO₂X peut conduire à un sel de sulfonimide symétrique (Rf¹SO₂)₂NM^{b}. Selon la stoechiométrie de la réaction, mécaniquement l'emploi d'au moins un équivalent de Rf¹SO₂X et d'au moins un équivalent de Rf²SO₂X peut conduire à un sel de sulfonimide symétrique (Rf¹SO₂)(Rf²SO₂)NM^{b} dans lequel Rf¹ et Rf² sont identiques, ou à un sel de sulfonimide dissymétrique : (Rf¹SO₂)(Rf²SO₂)NM^{b} dans lequel Rf¹ et Rf² sont différents.

Selon l'invention, selon sa stoechiométrie la réaction d'ammonolyse de la méthode iv) peut être réalisée en présence d'au moins un équivalent de Rf¹SO₂X, notamment Rf¹SO₂Cl, d'au moins un équivalent de Rf²SO₂NH₂ et d'au moins deux équivalents de base.

Ainsi, la méthode d'ammonolyse iv) peut conduire à un sel de sulfonimide symétrique (Rf¹SO₂)(Rf²SO₂)NM^{b} (ou (Rf¹SO₂)₂NM^{b}) dans lequel Rf¹ et Rf² sont identiques, ou à un sel de sulfonimide dissymétrique (Rf¹SO₂)(Rf²SO₂)NM^{b} dans lequel Rf¹ et Rf² sont différents.

Selon l'invention, l'étape d'ammonolyse réalisée dans les conditions de la méthode iii) peut conduire à un triflimidure symétrique de formule suivante [(Rf¹SO₂)₂N⁻, R'₃NH⁺] ou à un triflimidure dissymétrique de formule suivante (Rf¹SO₂)(Rf²SO₂)N⁻, R'₃NH⁺].

Selon l'invention, l'étape d'ammonolyse réalisée dans les conditions de la méthode iv) peut conduire à un triflimidure de formule (Rf¹SO₂)(Rf²SO₂)N⁻, R'₃NH⁺], ledit triflimidure étant symétrique (lorsque Rf¹ et Rf² sont identiques) ou dissymétrique (lorsque Rf¹ et Rf² sont différents).

Ainsi, de manière générale, l'étape d'ammonolyse selon l'invention, et quelle que soit la méthode utilisée, peut conduire à un triflimidure de formule [(Rf¹SO₂)(Rf²SO₂)N⁻, R'₃NH⁺] dans laquelle Rf¹ et Rf² sont identiques ou différents.

Selon l'invention, la réaction d'ammonolyse peut être réalisée dans un solvant organique ou en l'absence de solvant organique, et ce indépendamment de la méthode utilisée (méthode iii) ou iv)).

Selon un mode de réalisation, le solvant organique est avantageusement peu polaire du type solvants chlorés, nitriles, éthers ou solvants aromatiques. De préférence, le solvant est le dichlorométhane.

Selon l'invention, la réaction d'ammonolyse peut être réalisée en présence ou en l'absence d'un catalyseur.

Selon un mode de réalisation, lorsque l'étape d'ammonolyse est réalisée selon tes conditions de la méthode iii), un catalyseur, tel que la diméthylaminopyridine (DMAP), est utilisé.

Selon un mode de réalisation, lorsque l'étape d'ammonolyse est réalisée selon les conditions de la méthode iv), aucun catalyseur n'est utilisé.

Selon l'invention, la réaction d'ammonolyse peut être réalisée à pression atmosphérique ou sous pression, et notamment en phase au moins liquide et/ou gazeuse.

Selon un mode de réalisation, la réaction d'ammonolyse est réalisée dans un réacteur tabulaire sous pression.

Selon l'invention, NH₃, préférentiellement employé pour la mise en oeuvre de l'une des méthodes iii) ou iv), peut être sous forme gaz ou sous forme de solution aqueuse ou anhydre.

Selon un mode de réalisation, la base est une amine tertiaire de formule NR'₃, dans lequel R' représente un groupe alkyle, linéaire ou branché, contenant de 1 à 20 atomes de carbone. De préférence, l'amine tertiaire est choisie parmi la triéthylamine (TEA), la triisopropylaine, la dicyclohexyléthylamine, la diisopropyléthylamine (DIPEA). De préférence, la base utilisée est la diisopropyléthylamine ou la triéthylamine.

On utilise en particulier la diisopropyléthylamine lorsque Rf¹SO₂X est Rf¹SO₂Cl ou Rf¹SO₂Br, de préférence Rf¹SO₂Cl, et la triéthylamine lorsque Rf¹SO₂X est Rf¹SO₂F.

Selon un mode de réalisation, lorsque la réaction d'ammonolyse est réalisée avec la diisopropyléthylamine, l'étape d'ammonolyse conduit au complexe (Rf¹SO₂)₂N⁻,NEt(i-Pr)₂H⁺] ou au complexe (Rf¹SO₂)(Rf²SO₂)N⁻, NEt(i-Pr)₂H⁺].

Selon un autre mode de réalisation, lorsque la réaction d'ammonolyse est réalisée avec la triéthylamine, l'étape d'ammonolyse conduit au complexe suivant : [(Rf¹SO₂)₂N⁻, NEt₃H⁺] ou (Rf¹SO₂)(Rf²SO₂)N⁻, NEt₃H⁺].

Selon un mode de réalisation, la réaction d'ammonolyse est mise en oeuvre comme suit : une solution de Rf¹SO₂Cl et de Rf²SO₂Cl, dans le dichlorométhane, est ajoutée dans un milieu contenant la diisopropyléthylamine, un catalyseur, le dichlorométhane et de l'ammoniac. En particulier, la solution de Rf¹SO₂Cl et de Rf²SO₂Cl, est ajoutée à une température allant de -20°C à 10°C. Puis le milieu réactionnel est ensuite agité à température ambiante pendant au minimum une heure.

Selon l'invention, Rf²SO₂Cl peut être préparé selon les mêmes conditions que pour Rf¹SO₂Cl telles que décrites ci-dessus, ou selon les procédés connus de l'homme du métier.

Selon un autre mode de réalisation, la réaction d'ammonolyse est mise en oeuvre comme suit : Rf¹SO₂F et Rf²SO₂F, sont introduits avec de l'ammoniac, dans un milieu contenant de la triéthylamine, à une température allant de -80°C à -30°C, de préférence de -60°C à -40°C, et préférentiellement à environ -50°C. Puis le milieu réactionnel est ensuite agité à une température allant de 20°C à 80°C, de préférence de 50°C à 70°C, pendant le temps nécessaire pour que la réaction soit complète. En particulier, le milieu réactionnel est agité à 65°C pendant 24 heures.

Selon un mode de réalisation, l'étape d'ammonolyse comprend au moins une étape de lavage avec de l'eau. Si la réaction a été effectuée avec Rf¹SO₂F, et Rf²O₂F, un solvant organique, tel que le dichlorométhane, est ajouté dans le milieu pour l'étape de lavage avec de l'eau.

Selon l'invention, Rf²SO₂F peut être préparé selon les mêmes conditions que pour Rf¹SO₂F telles que décrites ci-dessus, ou selon les procédés connus de l'homme du métier.

Ainsi, l'étape d'ammonolyse peut conduire à un mélange biphasique M9, comprenant une phase organique O9 et une phase aqueuse A9.

En particulier, la phase organique O9 comprend le complexe (Rf¹SO₂)(Rf²SO₂)NH,R'₃N], tel que (Rf¹SO₂)(Rf²SO₂)NH,NEt(i-Pr) ou (Rf¹SO₂)(Rf²SO₂)NH,NEt₃ le dichlorométhane, ainsi que des impuretés organiques formées au cours des différentes étapes du procédé, telles que l'étape de sulfination b), d'oxydation c) et/ou d'ammonolyse d). En particulier, les impuretés organiques présentes dans la phase organique O9 sont les sels Rf¹SO₂NH₂,R'₃N et/ou (Rf²SO₂)NH₂,R'₃N, Rf¹SO₂H,R'₃N et/ou Rf²SO₂H,R'₃N et Rf¹SO₃H,R'₃N et/ou Rf²SO₃H,R'₃N. En particulier, lorsque la triéthylamine est utilisée, les impuretés organiques sont Rf¹SO₂NH₂,NEt₃, (Rf²SO₂)NH₂,NEt₃, Rf¹SO₂H,NEt₃ Rf²SO₂H,NEt₃, et Rf¹SO₃H,NEt₃ et Rf¹SO₃H,NEt₃. En particulier, lorsque la diisopropylamine est utilisée, les impuretés organiques sont Rf¹SO₂NH₂,NEt(i-Pr)₂, Rf²SO₂NH₂,NEt(i-Pr)₂, Rf¹SO₂H,NEt(i-Pr)₂, Rf²SO₂H,NEt(i-Pr)₂, et Rf¹SO₃H,NEt(i-Pr)₂, et Rf¹SO₃H,NEt(i-Pr)_{2.}

L'étape de lavage permet avantageusement de réduire la quantité de chlorures, de bromures ou de fluorures présents dans la phase organique O9.

Selon un mode de réalisation, la phase A9 comprend les sulfonylimidure d'ammonium, notamment les perfluoroalkylsulfonylimidure d'ammononium, formés durant l'étape d'ammonolyse, et les sels d'ammonium de fluor, brome ou chlore.

En particulier, la phase A9 comprend le chlorhydrate de diisopropylamine lorsque la diisopropylamine a été utilisée dans l'étape d'ammonolyse ou le ftuorhydrate de triéthylamine lorsque la triéthylamine a été utilisée.

Le procédé selon l'invention peut comprendre une étape de traitement de la phase aqueuse A9. En particulier, l'étape de traitement est réalisée en présence de soude à 30%. En particulier, cette étape permet de récupérer la base R'₃N, et notamment la diisopropylamine ou la triéthylamine, par neutralisation avec la soude du chlorhydrate de diisopropyléthylamine, ou du fluorhydrate de la triéthylamine, contenu dans la phase aqueuse A9. La base, notamment la diisopropylamine ou la triéthylamine, peut ainsi être avantageusement récupérée et recyclée dans le procédé de l'invention, et notamment à l'étape d'ammonolyse.

Selon un mode de réalisation, l'étape d'ammonolyse peut comprendre une étape de distillation du solvant de la réaction, notamment du dichlorométhane. En particulier, ledit solvant peut être recyclé dans le procédé, notamment dans l'étape d) d'ammonolyse.

### Etape e) d'acidification

Dans le cadre de l'invention, le procédé de préparation de (Rf¹SO₂)(Rf²SO₂)NM^{b}, et notamment de (CF₃SO₂)₂NLi peut comprendre une étape e) d'acidification, et notamment de la phase organique O9.

L'étape d'acidification peut être réalisée selon les procédés connus de l'homme du métier.

Selon un mode de réalisation, l'étape d'acidification e) est réalisée sur la phase organique 09 ayant subi une étape préalable de distillation du solvant organique.

Selon un mode de réalisation, l'étape d'acidification peut être réalisée en présence d'un acide choisi dans le groupe constitué de : l'acide chlorhydrique, l'acide sulfurique, l'acide phosphorique et l'acide nitrique. De préférence, l'étape d'acidification est réalisée avec l'acide sulfurique, notamment à 92%.

Selon un mode de réalisation, l'étape d'acidification permet de neutraliser le complexe (Rf¹SO₂)(Rf²SO₂)NH,NR'₃, tel que (Rf¹SO₂)(Rf²SO₂)NH,EtN(i-Pr)₂ ou ((Rf¹SO₂)(Rf²SO₂)NH,NEt₃, formé à l'issue de l'étape d'ammonolyse précédente, pour conduire au mélange M'1 tel que défini ci-dessus.

En particulier, le mélange M'1 comprend (Rf¹SO₂)(Rf²SO₂)NH, et des produits secondaires (impuretés organiques et minérales) tels que l'hydrogénosulfate de la base amine tertiaire, Rf¹SO₂H et/ou Rf²SO₂H, Rf¹SO₃H et/ou Rf²SO₃H, Rf¹SO₂NH₂ et/ou Rf²SO₂NH_{2,} HCl, HBr ou HF.

De préférence, l'hydrogénosulfate de la base amine tertiaire est l'hydrogénosulfate de la diisopropyléthylamine (H₂SO₄,NEt(i-Pr)₂) ou celui de la triéthylamine (H₂SO₄,NEt₃).

Selon le procédé de l'invention, le mélange M'1 peut être soumis à une étape de distillation telle que définie précédemment. En particulier, l'étape de distillation du mélange M'1 conduit au mélange M1.

Selon un mode de réalisation, le mélange M'1 ayant été soumis à une étape de distillation, comprend notamment l'hydrogénosulfate de diisopropyléthylammonium ou de triéthylammonium. Celui-ci peut être dilué dans l'eau et traité par de la soude aqueuse pour former respectivement la diisopropylamine ou la triéthylamine. En particulier, la diisopropylamine ou la triéthylamine récupérée peut être distillée sous forme de son azéotrope avec l'eau, pour former un mélange biphasique comprenant une phase aqueuse et une phase organique. En particulier, la phase organique comprend la diisopropylamine ou la triéthylamine quasiment pure, qui est recyclée dans le procédé, et notamment dans l'étape d'ammonolyse d).

### Etape f) d'oxydation

Le procédé selon l'invention peut également comprendre une étape d'oxydation du mélange M1 telle que définie précédemment. En particulier, l'étape d'oxydation f) du mélange M1 conduit au mélange M2, ces deux mélanges étant tels que définis ci-dessus.

Selon l'invention, l'étape f) d'oxydation correspond à l'étape i) telle que décrite précédemment.

Conformément à l'invention, le procédé de l'invention comprend une étape ii) de distillation du mélange M2, ladite étape de distillation étant telle que définie précédemment pour conduire à (Rf¹SO₂)(Rf²SO₂)NH aqueux.

### Etape g) de neutralisation

Le procédé selon l'invention peut également comprendre à l'issue de l'étape ii) de distillation, une étape de neutralisation de (Rf¹SO₂)(Rf²SO₂)NH aqueux.

L'étape de neutralisation peut être effectuée dans les conditions connues de l'homme du métier.

Selon un mode de réalisation, l'étape de neutralisation est effectuée en présence d'une base alcaline, pour conduire à une solution aqueuse de (Rf¹SO₂)(Rf²SO₂)NM^{b}.

Selon un mode de réalisation, la base alcaline est une base de lithium, de potassium, de sodium, de césium.

En particulier, la base alcaline est une base lithiée, choisie dans le groupe constitué de : LiOH, LiH, LiOH.H₂O ou Li₂CO₃. De préférence, la base utilisée est LiOH.H₂O.

Selon un mode de réalisation, l'étape de neutralisation de (Rf¹SO₂)(Rf²SO₂)NH aqueux est effectuée à une température inférieure à 35°C.

Selon un mode de réalisation, (Rf¹SO₂)(Rf²SO₂)NM^{b}, et notamment (CF₃SO₂)₂NLi est obtenu sous forme de solution aqueuse.

### Etape h) de séchage

Le procédé selon l'invention peut également comprendre une étape h) de séchage de la solution aqueuse de (Rf¹SO₂)(Rf²SO₂)NM^{b}, et notamment (Rf¹SO₂)₂Li telle que définie ci-dessus.

L'étape de séchage peut être effectuée dans les conditions connues de l'homme du métier.

Selon un mode de réalisation, le procédé de préparation (Rf¹SO₂)(Rf²SO₂)NM^{b} comprend une étape de séchage de la solution aqueuse (Rf¹SO₂)(Rf²SO₂)NM^{b}. Ainsi, (Rf¹SO₂)(Rf²SO₂)NM^{b} est séché préférentiellement par atomisation. Un séchage plus poussé peut être obtenu par évaporation à l'évaporateur rotatif, sous vide, puis dans une étuve à 100°C sous vide (5-25 mbar). L'étape de séchage permet de conduire à (Rf¹SO₂)(Rf²SO₂)NM^{b} sous forme solide.

Selon un mode de réalisation, (Rf¹SO₂)(Rf²SO₂)NM^{b} est obtenu à l'issue de l'étape de séchage, avec une pureté supérieure à 99%, et de préférence supérieure à 99,5%.

Il a été avantageusement montré que le procédé de l'invention permet de préparer une solution de (Rf¹SO₂)(Rf²SO₂)NH avec une pureté élevée, notamment supérieure à 99,5%. Ainsi, la solution aqueuse de (Rf¹SO₂)(Rf²SO₂)NH permet avantageusement la préparation de (Rf¹SO₂(Rf²SO₂)NM^{b}, et notamment (Rf¹SO₂)(Rf²SO₂)NLi, avec une pureté élevée, notamment supérieure à 99,5%.

Le procédé selon l'invention permet avantageusement de préparer une solution aqueuse de (Rf¹SO₂R)(Rf²SO₂)NH dépourvue d'impuretés organiques telles que Rf¹SO₂NH₂ et/ou Rf²SO₂NH₂. De plus, le procédé selon l'invention permet avantageusement de recycler Rf¹SO₂NH₂ et/ou Rf²SO₂NH₂ dans le procédé.

Le procédé selon l'invention permet avantageusement de préparer (Rf¹SO₂)(Rf²SO₂)NM^{b} pur et ne présentant pas de coloration.

Le procédé selon l'invention permet avantageusement d'éviter l'emploi de solvants toxiques et inflammables.

Les exemples suivants permettent d'illustrer l'invention.

### Exemples :

### Abréviations

- [(CF₃SO₂)₂NH: : bis-trifluorométhanesulfonimide (TFSIH)
- [(CF₃SO₂)₂NLi]: : bis-trifluorométhanesulfonimidure de lithium (LiTFSI)
- CF₃SO₂NH₂: : trifluorosulfonamide (TFSAH)
- CF₃SO₂F: : Fluorure de trifluorométhanesulfonyle (TFSF)
- CF₃SO₂Cl: : chlorure de trifluorométhanesulfonyle (TFSCl)
- CF₃SO₂Br: : bromure de trifluorométhanesulfonyle (TFSBr)
- CF₃CO₂K: : trifluoroacétate de potassium (TFAK)
- CF₃SO₂K: : triflinate de potassium (TFSK)
- CF₃CO₂H: : acide triftuoroacétique (TFA)
- CF₃SO₂H: : acide triflinique (TFSH)
- CF₃SO₃H: : acide trifluorométhanesulfonique (acide triflique, TA)
- CH₃SO₂Cl: : chlorure de mésyle (MsCl)
- CH₃SO₂F: : fluorure de mésyle (MsF)
- DIPEA: : diisopropyléthylamine
- TEA: : triéthylamine

### Exemple 1 :

### Etape a :

Le TFAK (1,04kG) a été préparé par neutralisation d'une solution aqueuse de potasse (en conservant une température inférieure à 25°C) suivie de l'évaporation de la solution correspondante et séchage des cristaux obtenus. TFAK a été obtenu avec une pureté supérieure à 99%.

### Etape b:

Une solution de TFAK (0,913 kg, soit 6 moles) dans le DMF (5,22kg) a été introduite dans un autoclave de 15L, préalablement lavé et séché. Le SO₂ a alors été ajouté à la solution TFAK/DMF par bullage, et la quantité exacte introduite (0,768kg, soit SO₂/TFAK molaire ∼2 l) a été déterminée par pesée. Le réacteur a ensuite été fermé et chauffé sous agitation à 140°C pendant 4 à 5h. Après retour à l'ambiante, le réacteur a été dégazé, et son contenu a été transféré dans une recette en verre de 10L. Après dilution adéquate, l'analyse par chromatographie ionique de la masse réactionnelle a conduit aux résultats suivants :
- Bilan pondéral : 97% (poids recueilli : 6,7kg)
- Conversion du TFAK (molaire): 64 % ± 2.5% (TFAK dosé dans le milieu rèactionnel : 0,326kg)
- Rendement en TFSK (molaire): 50 % ± 2.5% (TFSK dosé dans le milieu réactionnel : 0,517kg)

Le brut réactionnel (mélange de TFAK et TFSK) précédent (6,5kg) a ensuite été concentré sous vide pour conduire à 4,1kg de DMF, recyclé à l'opération suivante, et à un culot (2,02kg) vidangé vers l'étape suivante.

### Etape c:

De l'eau (2kg) a été additionnée au brut réactionnel concentré de l'étape (b) précédente (environ 2kg), puis la solution résultante a été extraite par du dichlorométhane pour conduire à :
- une phase aqueuse (2,4kg) contenant 0,236kg de TFAK et 0.471kg de TFSK et moins de 1000ppm de DMF ; et
- une phase organique composée essentiellement de dichlorométhane et de DMF, dont la distillation a permis de récupérer successivement le dichlorométhane puis le DMF qui sont recyclés dans le procédé.

La phase aqueuse précédente a été introduite dans un réacteur en verre de 3L, refroidie à 0°C, puis du dichlore gazeux a été introduit. Le TFSCl a été récupéré après décantation du milieu réactionnel, puis purifié par distillation à pression atmosphérique pour conduire au TFSCl pur (0,422kg, RR de 91% en molaire).

La phase aqueuse obtenue, contenant essentiellement du TFAK et du KCI, a été extraite par un mélange DIPE (diisopropyl éther) / HCl pour conduire après décantation à une phase organique constituée de TFA, d'eau et de DIPE. La phase organique obtenue a ensuite été contre-extraite par de la potasse aqueuse pour conduire à une solution aqueuse de TFAK, recyclée dans le procédé.

### Etape d:

Dans un réacteur en verre de 5L ont été introduits: 323g (2,5mol) de DIPEA (diisopropyléthyl amine), 15,3g (0,125mol) de DMAP (4-diméthylaminopyridine) et 0,8L de dichlorométhane. Le milieu réactionnel correspondant a été refroidi à -15°C et l'ammoniac gaz (25,5, 1,5mol) a été introduit. La quantité exacte chargée a été déterminée par pesée. La solution de CF₃SO₂Cl (674g à 50% p/p dans le CH₂Cl₂ (2 mol)) a ensuite été injectée *via* un pousse-seringue en maintenant la température de la masse réactionnelle autour de 0°C. La température a ensuite été remontée à 20°C. La masse réactionnelle a alors été lavée à l'eau, pour stabiliser la masse réactionnelle et avoir deux phases homogènes facilement analysables. Environ 2,1kg de phase organique et 0,540kg de phase aqueuse ont été récupérés.

### Etape e:

La phase organique de l'étape (d) précédente a été concentrée par distillation du dichlorométhane (1,4kg) qui a ensuite été recyclé dans le procédé. Puis le résidu de distillation a été traité par de l'acide sulfurique concentré (0,920kg). Une distillation sous pression réduite a permis d'isoler le TFSIH "brut" (0,246kg) (correspondant au mélange M1 avec les acides triflinique (3,8%mol) et triflique (3,5%mol) et la trifluorosulfonamide (0.7%mol)) avec une pureté molaire de 92% et une pureté massique d'environ 96%.

### Etape f:

L'acide triflinique TFSH contenu dans le TFSIH "brut" (1,9% p/p) a été oxydé en acide trfflique (TA) par l'eau oxygénée 30% de la manière suivante : le TFSIH "brut" de l'étape précédente e, a été porté à 80°C, puis l'eau oxygénée à 30% (3,15g) a été ajoutée. Les analyses ont montré que pratiquement tout le TFSH a été converti (teneur : 0,04%p/p) en acide triflique (teneur : 3,4%p/p) alors que le TFSIH (teneur : 96%p/p) et la TFSAH (teneur : 0,4%p/p) n'ont pas été affectés.

### Etape g:

Le milieu réactionnel de l'étape précédente f (0,246kg) (mélange M2) a été additionné d'eau déminéralisée (0,2kg), puis introduit dans le bouilleur d'un appareil de distillation surmonté d'une colonne de distillation comportant 14 plateaux physiques. Le milieu réactionnel a été porté à 90°C sous 20 à 60mbar jusqu'à reflux total. L'eau a ensuite été soutirée avec un taux de reflux de 5 à 15, et une température en tête de colonne de 20°C à 45°C. Lorsque la température en milieu de colonne a atteint une température de 20°C à 55°C, la fraction de tête a été soutirée dans une autre recette avec un taux de reflux compris de 5 à 15 et une température en tête de colonne comprise de 40°C à 100°C. Lorsque la quantité des fractions de têtes a atteint environ 90% de l'eau contenue dans le TFSIH de départ, les conduites de soutirage et de reflux ont été portées à environ 60°C, et la fraction de tête a été recueillie jusqu'à ce que la teneur en fluorures dans le distillat soit inférieure à 20ppm. La pression a alors été abaissée à une pression comprise de 5 à 15mbar et le milieu réactionnel a été porté à reflux total. La fraction de coeur a alors été recueillie avec un taux de reflux compris de 5 à 15 et une température en tête de colonne comprise de 50°C à 80°C. Le soutirage de la fraction de coeur a été arrêté lorsque la température du bouilleur a atteint une température comprise de 110°C à 130°C.

Après analyses, les fractions de coeur ont été rassemblées et diluées à l'eau pour conduire à une solution aqueuse de TFSIH "pur" (∼70%p/p, 0,315kg) contenant moins de 20ppm de fluorures, chlorures et sulfates et moins de 500 ppm d'impuretés organiques cumulées, exprimées par rapport au produit anhydre (TA : < 50ppm, TFSH et TFSAH < 100ppm).

### Etape h:

Dans un réacteur en verre de 1L double-enveloppé, le milieu réactionnel de l'étape précédente g a été porté à 20°C. La lithine (33g), sous forme solide de monohydrate (LiOH, H₂O) a été additionnée jusqu'à neutralité de la solution. La température du milieu réactionnel a été maintenue en dessous de 35°C, et le pH de la solution finale a été ajusté à la neutralité.

### Etape i:

La solution aqueuse de l'étape précédente h a été concentrée à l'évaporateur rotatif sous vide, puis séchée à l'étuve sous vide, pour conduire à une poudre blanche de LiTFSI (0,225kg). Les analyses du LiTFSI obtenu ont montré qu'il contient moins de 100ppm d'eau, moins de 20ppm de fluorures et sulfates, moins de 15ppm de chlorures, moins de 10ppm de sodium, moins de 5 ppm de calcium, potassium et silicium, moins de 2 ppm de fer et moins de 1 ppm de nickel, bore, aluminium et magnésium.

### Etape i:

Les eaux de lavage du milieu réactionnel de l'étape d (0,540kg) ont été traitées pour conduire à la DIPEA (0,310kg) qui a été recyclée dans le procédé.

Le complexe TFSIH-DIPEA a été extrait des culots sulfuriques de rétape e (1,5kg) par le dichlorométhane. La fraction organique a ensuite été recyclée à l'étape de la concentration dichlorométhane de l'étape e. La DIPEA a été distillée sous forme de son azéotrope avec l'eau qui décante en une couche organique (0,16kg) et une couche aqueuse (0,315kg). La couche organique, constituée de DIPEA quasi pure, a pu être recyclée dans le procédé.

### Exemple 2 :

### Etapes a' et b' : identiques aux étapes (a) et (b) de l'exemple 1

### Etape c':

Une phase aqueuse (0.5kG), obtenue dans les mêmes conditions que celles décrites pour l'étape c précédente avant oxydation, a été introduite dans un réacteur en verre de 1L double-enveloppé. Le milieu réactionnel a été refroidi à -5°C, puis du difluor gazeux dilué (5% v/v dans l'azote) a été introduit en masse tout en maintenant une température inférieure à -5°C. Le fluorure de triftyte gazeux formé a été condensé dans une recette en verre refroidie par un mélange acétone/carboglace (∼-50°C). Le TFSF récupéré (0,0450kg, RR molaire 52%) a été transféré dans un autoclave en inox 316L de 0,1L de volume utile.

La phase aqueuse résultante, contenant essentiellement du TFAK et du KF, a été extraite par un mélange DIPE (diisopropyl éther) / HCl pour conduire après décantation à une phase organique constituée de TFA, d'eau et de DIPE. La phase organique obtenue a ensuite été contre-extraite par de la potasse aqueuse pour conduire à une solution aqueuse de TFAK, recyclée dans le procédé.

### Etape d' :

Dans un autoclave en alliage Hastelloy® C276 de 25ml ont été introduits 8g (0,079mol) de TEA (triéthylamine). L'autoclave a alors été refroidi à environ -50°C, puis 2,5g (16mmol) de fluorure de triflyle obtenu précédemment (étapes c') et 0,114g (6,7mmol) d'ammoniac ont été introduits. L'autoclave a alors été fermé et porté à 65°C pendant 24h. Après retour à 20°C, la masse réactionnelle a été diluée par du dichlorométhane et lavée à l'eau, pour avoir deux phases homogènes facilement analysables. Les rendements molaires calculés sont de :
- TFSI⁻ : 74%
- TFS⁻ : 6%
- TA⁻ : 10%
- TFSA⁻ : 4%

**Etapes e' à i' :** Le traitement aval de la phase organique dans des conditions analogues à celles décrites dans les étapes (e) à (1) de l'exemple 1 permet d'isoler du LiTFSI de haute pureté (1,5g, rendement molaire : 78%).

### Exemple 3 :

### Etapes a"

Dans un réacteur en verre de 1L double-enveloppé, de l'eau (650mL) et du fluorure de potassium (175g) ont été introduits. Le milieu réactionnel a été porté à 10-20°C, et le chlorure de mésyle (MsCl : 350g) a été additionné en maintenant le milieu réactionnel à une température inférieure à 30°C. Le fluorure de mésyle (MsF) a été recueilli par décantation et purifié par distillation. Au final 275g de MsF ont été obtenus (94%).

### Etape b":

Dans une cellule électrochimique de 2L munie d'électrodes en nickel d'une surface anodique totale de 1050cm² ont été introduits :
- HF : 0,6L
- CH₃SO₂F : 100g (sur 24h)

Le courant a été ajusté entre 4,5 et 6V avec une densité de courant de 9 à 12.5mA/cm², Les gaz produits ont été lavés à l'eau puis à l'acide sulfurique concentré et le fluorure de triflyle a été recueilli dans un piège refroidi à -70°C. Le rendement global est d'environ 80%.

**Etapes d" à i": identiques aux étapes (d') à (i') de l'exemple 2**

## Revendications

1. Procédé de préparation d'une solution aqueuse d'un composé sulfonimide de formule (Rf¹-SO₂)(Rf²-SO₂)NH à partir d'un mélange M1 comprenant (Rf¹-SO₂)(Rf²-SO₂)NH et Rf¹SO₂H et/ou Rf²SO₂H, Rf¹ et Rf² étant indépendamment l'un de l'autre choisi dans le groupe constitué de : l'atome de fluor et des groupes ayant de 1 à 10 atomes de carbone étant choisis parmi les groupes perfluoroalkyles et fluoroalkyles, **caractérisé en ce qu'**il comprend :
i) une étape d'oxydation dudit mélange M1 par un agent oxydant, pour obtenir un mélange M2 comprenant (Rf¹-SO₂)(Rf²-SO₂)NH et Rf¹SO₃H et/ou Rf²SO₃H; et
ii) une étape de distillation du mélange M2, en milieu aqueux pour séparer (Rf¹-SO₂)(Rf²-SO₂)NH de Rf¹SO₃H et/ou de Rf²SO₃H.

2. Procédé selon la revendication 1, dans lequel le mélange M1 comprend (Rf¹-SO₂)(Rf²-SO₂)NH, Rf¹SO₂H et/ou Rf²SO₂H, Rf¹SO₂NH₂ et/ou Rf²SO₂NH₂.

3. Procédé selon l'une quelconque des revendications 1 ou 2, dans lequel l'étape de distillation est effectuée dans appareil de distillation comprenant un bouilleur, dont la température du bouilleur va de 50°C à 300°C.

4. Procédé selon l'une quelconque des revendications 1 à 3, dans lequel l'étape de distillation est effectuée dans un appareil de distillation, comprenant une colonne de distillation dont la température en tête de colonne va de 20°C à 180°C.

5. Procédé selon l'une quelconque des revendications 1 à 4, dans lequel l'étape de distillation est effectuée dans un appareil de distillation comprenant une colonne de distillation dont la pression dans ladite colonne de distillation est comprise de 1 à 1000 mbar, ladite colonne comprenant notamment de 2 à 40 plateaux théoriques.

6. Procédé selon l'une quelconque des revendications 1 à 5, dans lequel le mélange M1 est obtenu par distillation d'un mélange M'1, comprenant (Rf¹-SO₂)(Rf²-SO₂)NH et Rf¹SO₂H et/ou Rf²SO₂H.

7. Procédé selon la revendication 6 dans lequel le sulfonimide (Rf¹-SO₂)(Rf²-SO₂)NH présent dans le mélange M'1 est obtenu par une étape d'acidification d'une phase organique comprenant un complexe (Rf¹-SO₂)(Rf²-SO₂)NH,NR'₃, R' représentant un groupe alkyle, linéaire ou branché, contenant de 1 à 20 atomes de carbone.

8. Procédé selon la revendication 7, dans lequel le complexe (Rf¹-SO₂)(Rf²-SO₂)NH,NR'₃ est obtenu par une étape d'ammonolyse de Rf¹SO₂X et de Rf²SO₂X, X représentant Cl, Br ou F, Rf¹ et Rf² étant tels que définis dans la revendication 1.

9. Procédé selon la revendication 8, dans lequel Rf¹SO₂X est obtenu par une étape d'oxydation d'un mélange M3 comprenant Rf¹CO₂M^{a}, M^{a} représentant un métal alcalin, et Rf¹SO₂M^{a}, pour obtenir un mélange biphasique M4 ledit mélange M4 comprenant Rf¹SO₂X et Rf¹CO₂M^{a}.

10. Procédé selon la revendication 9, dans lequel Rf¹SO₂X, avec X = F, est obtenu par électrofluoration de R^{h}SO₂F, R^{h} représentant une chaîne hydrocarbonée identique à celle de Rf¹, ladite chaîne comprenant des atomes d'hydrogène à la place des atomes de fluor, et R^{h}SO₂F étant éventuellement obtenu à partir de R^{h}SO₂W, avec W = Cl, Br, ou I.

11. Procédé selon la revendication 9, dans lequel le mélange M3 est obtenu par :
- une étape de sulfination de Rf¹CO₂M^{a}, et notamment de CF₃CO₂K, dans un solvant organique, pour conduire à un mélange M comprenant Rf¹SO₂M^{a}, et notamment CF₃SO₂K, et ledit solvant organique ; puis
- une étape de distillation dudit solvant organique du mélange M, pour obtenir le mélange M sans ledit solvant; puis
- une étape de séparation des sels du mélange M issu de l'étape de distillation,
le cas échéant, Rf¹CO₂M^{a} étant obtenu par une étape de salification de Rf¹CO₂H, de préférence de CF₃CO₂H.

12. Procédé de préparation de (Rf¹-SO₂)(Rf²-SO₂)NM^{b}, M^{b} représentant un métal alcalin, comprenant :
- la préparation d'une solution aqueuse de (Rf¹-SO₂)(Rf²-SO₂)NH selon le procédé tel que défini selon les revendications 1 à 11,
- une étape de neutralisation de (Rf¹-SO₂)(Rf²-SO₂)NH, en présence d'une base de métal alcalin,
- le cas échéant suivi d'une étape de séchage.

13. Procédé de préparation de (Rf¹-SO₂)(Rf²-SO₂)NM^{b} comprenant les étapes suivantes :
- une étape a) de salification de Rf¹CO₂H en Rf¹CO₂M^{a} ;
- une étape b) de sulfination de Rf¹CO₂M^{a} en Rf¹SO₂M^{a} ;
- une étape c) d'oxydation de Rf¹SO₂M^{a} en Rf¹SO₂X ;
- une étape d) d'ammonolyse de Rf¹SO₂X en (Rf¹SO₂)(Rf²-SO₂)NH,NR'₃;
- une étape e) d'acidification de (Rf¹SO₂)(Rf²SO₂)NH,NR'₃ en (Rf¹SO₂)(Rf²SO₂)NH;
- une étape g) de neutralisation, par une base de métal alcalin, de (Rf¹SO₂)(Rf²SO₂)NH en (Rf¹-SO₂)(Rf²-SO₂)NM^{b}; et
- le cas échéant une étape h) de séchage de (Rf¹SO₂)(Rf²SO₂)NM^{b},
dans lequel (Rf¹-SO₂)(Rf²-SO₂)NH, obtenu à l'issue de l'étape e), est sous forme d'un mélange M1 comprenant (Rf¹-SO₂)(Rf²-SO₂)NH et Rf¹SO₂H et/ou Rf²SO₂H, et (Rf¹-SO₂)(Rf²-SO₂)NH est purifié entre l'étape e) et l'étape g) par la mise en oeuvre du procédé tel que défini dans les revendications 1 à 11; et M^{a} et M^{b} représentant, indépendamment l'un de l'autre, un métal alcalin.

14. Procédé de préparation de (Rf¹-SO₂)(Rf²-SO₂)NM^{b} comprenant les étapes suivantes :
- une étape b') de transformation de R^{h}SO₂W, avec W représentant Br, Cl ou I, en R^{h}SO₂F ;
- une étape c') d'électrofluoration de R^{h}SO₂F en Rf¹SO₂F ;
- une étape d) d'ammonolyse de Rf¹SO₂F en (Rf¹SO₂)(Rf²-SO₂)NH,NR'₃;
- une étape e) d'acidification de (Rf¹SO₂)(Rf²SO₂)NH,NR'₃ en (Rf¹SO₂)(Rf²SO₂)NH;
- une étape g) de neutralisation, par une base de métal alcalin, de (Rf¹SO₂)(Rf²SO₂)NH en (Rf¹-SO₂)(Rf²-SO₂)NM^{b}; et
- le cas échéant une étape h) de séchage de (Rf¹SO₂)(Rf²SO₂)NM^{b},
dans lequel (Rf¹-SO₂)(Rf²-SO₂)NH, obtenu à l'issue de l'étape e), est sous forme d'un mélange M1 comprenant (Rf¹-SO₂)(Rf²-SO₂)NH et Rf¹SO₂H et/ou Rf²SO₂H, et (Rf¹-SO₂)(Rf²-SO₂)NH est purifié entre l'étape e) et l'étape g) par la mise en oeuvre du procédé tel que défini dans les revendications 1 à 11; et M^{b} représentant un métal alcalin, et R^{h} représentant une chaîne hydrocarbonée identique à celle de Rf¹, ladite chaîne comprenant des atomes d'hydrogène à la place des atomes de fluor.

## Patentansprüche

1. Verfahren zur Herstellung einer wässrigen Lösung einer Sulfonimidverbindung der Formel (Rf¹-SO₂)(Rf²-SO₂) NH aus einem Gemisch M1, umfassend (Rf¹-SO₂)(Rf²-SO₂)NH und Rf¹SO₂H und/oder Rf²SO₂H, wobei Rf¹ und Rf² unabhängig voneinander ausgewählt sind aus der Gruppe bestehend aus: dem Fluoratom und Gruppen mit 1 bis 10 Kohlenstoffatomen, ausgewählt aus Perfluoralkyl- und Fluoralkylgruppen, **dadurch gekennzeichnet, dass** es Folgendes umfasst:
i) einen Schritt zur Oxidation des Gemischs M1 mit einem Oxidationsmittel, um ein Gemisch M2, umfassend (Rf¹-SO₂)(Rf²-SO₂) NH und Rf¹SO₃H und/oder Rf²SO₃H, zu erhalten; und
ii) einen Schritt zur Destillation des Gemischs M2 in wässrigem Medium, um (Rf¹-SO₂)(Rf²-SO₂)NH von Rf¹SO₃H und/oder von Rf²SO₃H zu trennen.

2. Verfahren nach Anspruch 1, wobei das Gemisch M1 (Rf¹-SO₂)(Rf²-SO₂)NH, Rf¹SO₂H und/oder Rf²SO₂H, Rf¹SO₂NH₂ und/oder Rf²SO₂NH₂ umfasst.

3. Verfahren nach einem der Ansprüche 1 oder 2, wobei der Schritt zur Destillation in einem Destillationsapparat durchgeführt wird, umfassend einen Siedekessel, wobei die Temperatur des Siedekessels im Bereich von 50 °C bis 300 °C liegt.

4. Verfahren nach einem der Ansprüche 1 bis 3, wobei der Schritt zur Destillation in einem Destillationsapparat durchgeführt wird, umfassend eine Destillationskolonne, wobei die Temperatur am Kopf der Kolonne im Bereich von 20 °C bis 180 °C liegt.

5. Verfahren nach einem der Ansprüche 1 bis 4, wobei der Schritt zur Destillation in einem Destillationsapparat durchgeführt wird, umfassend eine Destillationskolonne, wobei der Druck in der Destillationskolonne im Bereich zwischen 1 und 1.000 mbar liegt, wobei die Kolonne insbesondere 2 bis 40 theoretische Böden umfasst.

6. Verfahren nach einem der Ansprüche 1 bis 5, wobei das Gemisch M1 durch Destillation eines Gemischs M'1, umfassend (Rf¹-SO₂)(Rf²-SO₂)NH und Rf¹SO₂H und/oder Rf²SO₂H, erhalten wird.

7. Verfahren nach Anspruch 6, wobei das Sulfonimid (Rf¹-SO₂)(Rf²-SO₂)NH, das in dem Gemisch M'1 enthalten ist, durch einen Schritt zur Ansäuerung einer organischen Phase erhalten wird, umfassend einen Komplex (Rf¹-SO₂)(Rf²-SO₂) NH, NR'₃, wobei R' für eine lineare oder verzweigte Alkylgruppe, enthaltend 1 bis 20 Kohlenstoffatome, steht.

8. Verfahren nach Anspruch 7, wobei der Komplex (Rf¹-SO₂)(Rf²-SO₂)NH,NR'₃ durch einen Schritt zur Ammonolyse von Rf¹SO₂X und von Rf²SO₂X erhalten wird, wobei X für Cl, Br oder F steht, wobei Rf¹ und Rf² so sind, wie in Anspruch 1 definiert.

9. Verfahren nach Anspruch 8, wobei Rf¹SO₂X durch einen Schritt zur Oxidation eines Gemischs M3, umfassend Rf¹CO₂M^{a}, wobei M^{a} für ein Alkalimetall steht, und Rf¹SO₂M^{a}, erhalten wird, um ein zweiphasiges Gemisch M4 zu erhalten, wobei das Gemisch M4 Rf¹SO₂X und Rf¹CO₂M^{a} umfasst.

10. Verfahren nach Anspruch 9, wobei Rf¹SO₂X, mit X = F, durch Elektrofluorierung von R^{h}SO₂F erhalten wird, wobei R^{h} für eine Kohlenwasserstoffkette gleich der von Rf¹ steht, wobei die Kette Wasserstoffatome anstelle der Fluoratome umfasst, und wobei R^{h}SO₂F gegebenenfalls aus R^{h}SO₂W, mit W = Cl, Br oder I, erhalten wird.

11. Verfahren nach Anspruch 9, wobei das Gemisch M3 erhalten wird durch:
- einen Schritt zur Sulfinierung von Rf¹CO₂M^{a}, und insbesondere von CF₃CO₂K, in einem organischen Lösungsmittel, um ein Gemisch M zu ergeben, umfassend Rf¹SO₂M^{a} und insbesondere CF₃SO₂K und das organische Lösungsmittel; dann
- einen Schritt zur Destillation des organischen Lösungsmittels des Gemischs M, um das Gemisch M ohne das Lösungsmittel zu erhalten; dann
- einen Schritt zur Trennung der Salze des Gemischs M aus dem Destillationsschritt, wobei Rf¹CO₂M^{a} gegebenenfalls durch einen Schritt zur Salzbildung von Rf¹CO₂H, vorzugsweise von CF₃CO₂H, erhalten wird.

12. Verfahren zur Herstellung von (Rf¹-SO₂)(Rf²-SO₂)NM^{b}, wobei M^{b} für ein Alkalimetall steht, umfassend:
- die Herstellung einer wässrigen Lösung von (Rf¹-SO₂)(Rf²-SO₂)NH gemäß dem Verfahren wie gemäß den Ansprüchen 1 bis 11 definiert,
- einen Schritt zur Neutralisierung von (Rf¹-SO₂)(Rf²-SO₂)NH in Gegenwart einer Alkalimetallbase,
- gegebenenfalls gefolgt von einem Schritt zur Trocknung.

13. Verfahren zur Herstellung von (Rf¹-SO₂)(Rf²-SO₂)NM^{b}, das die folgenden Schritte umfasst:
- einen Schritt a) zur Salzbildung von Rf¹CO₂H zu Rf¹CO₂M^{a} ;
- einen Schritt b) zur Salzbildung von Rf¹CO₂M^{a} zu Rf¹SO₂M^{a} ;
- einen Schritt c) zur Oxidation von Rf¹SO₂M^{a} zu Rf¹SO₂X ;
- einen Schritt d) zur Ammonolyse von Rf¹SO₂X zu (Rf¹SO₂)(Rf²-SO₂)NH,NR'₃;
- einen Schritt e) zur Ansäuerung von (Rf¹SO₂)(Rf²SO₂)NH,NR'₃ zu (Rf¹SO₂)(Rf²SO₂)NH;
- einen Schritt g) zur Neutralisation, durch eine Alkalimetallbase, von (Rf¹SO₂)(Rf²SO₂)NH zu (Rf¹-SO₂)(Rf²-SO₂)NM^{b}; und
- gegebenenfalls einen Schritt h) zur Trocknung von (Rf¹SO₂)(Rf²SO₂)NM^{b},
wobei (Rf¹-SO₂)(Rf²-SO₂)NH, erhalten am Ende von Schritt e), in Form eines Gemischs M1 vorliegt, umfassend (Rf¹-SO₂)(Rf²-SO₂)NH und Rf¹SO₂H und/oder Rf²SO₂H, und (Rf¹SO₂)(Rf²-SO₂)NH zwischen Schritt e) und Schritt g) gereinigt wird, indem das Verfahren wie in den Ansprüchen 1 bis 11 definiert durchgeführt wird; und M^{a} und M^{b} unabhängig voneinander für ein Alkalimetall stehen.

14. Verfahren zur Herstellung von (Rf¹-SO₂)(Rf²-SO₂)NM^{b}, das die folgenden Schritte umfasst:
- einen Schritt b') zur Umwandlung von R^{h}SO₂W, wobei W für Br, Cl oder I steht, zu R^{h}SO₂F;
- einen Schritt c') zur Elektrofluorierung von R^{h}SO₂F zu Rf¹SO₂F;
- einen Schritt d) zur Ammonolyse von Rf¹SO₂F zu (Rf¹SO₂)(Rf²-SO₂)NH,NR'₃;
- einen Schritt e) zur Ansäuerung von (Rf¹SO₂)(Rf²SO₂)NH,NR'₃ zu (Rf¹SO₂)(Rf²SO₂)NH;
- einen Schritt g) zur Neutralisation, durch eine Alkalimetallbase, von (Rf¹SO₂)(Rf²SO₂)NH zu (Rf¹-SO₂)(Rf²-SO₂)NM^{b}; und
- gegebenenfalls einen Schritt h) zur Trocknung von (Rf¹SO₂)(Rf²SO₂)NM^{b},
wobei (Rf¹-SO₂)(Rf²-SO₂)NH, erhalten am Ende von Schritt e), in Form eines Gemischs M1 vorliegt, umfassend (Rf¹-SO₂)(Rf²-SO₂)NH und Rf¹SO₂H und/oder Rf²SO₂H, und (Rf¹SO₂)(Rf²-SO₂)NH zwischen Schritt e) und Schritt g) gereinigt wird, indem das Verfahren wie in den Ansprüchen 1 bis 11 definiert durchgeführt wird; und M^{b} für ein Alkalimetall steht, und R^{h} für eine Kohlenwasserstoffkette gleich der von Rf¹ steht, wobei die Kette Wasserstoffatome anstelle von Fluoratomen umfasst.

## Claims

1. Process for preparing an aqueous solution of sulfonimide compound of formula (Rf¹-SO₂)(Rf²-SO₂)NH starting with a mixture M1 comprising (Rf¹-SO₂)(Rf²-SO₂)NH and Rf¹SO₂H and/or Rf²SO₂H, Rf¹ and Rf² being chosen, independently of each other, from the group consisting of: a fluorine atom and groups containing from 1 to 10 carbon atoms being chosen from perfluoroalkyl and fluoroalkyl groups, **characterized in that** it comprises:
i) a step of oxidizing said mixture M1 with an oxidizing agent, to obtain a mixture M2 comprising (Rf¹-SO₂)(Rf²-SO₂)NH and Rf¹SO₃H and/or Rf²SO₃H; and
ii) a step of distilling the mixture M2, in aqueous medium, to separate (Rf¹-SO₂)(Rf²-SO₂) NH from Rf¹SO₃H and/or from Rf²SO₃H.

2. Process according to Claim 1, **characterized in that** the mixture M1 comprises (Rf¹-SO₂)(Rf²-SO₂)NH, Rf¹SO₂H and/or Rf²SO₂H, Rf¹SO₂NH₂ and/or Rf²SO₂NH₂.

3. Process according to either of Claims 1 and 2, in which the distillation step is performed in distillation apparatus comprising a boiler in which the boiler temperature ranges from 50°C to 300°C.

4. Process according to any one of Claims 1 to 3, in which the distillation step is performed in a distillation apparatus comprising a distillation column in which the temperature at the top of the column ranges from 20°C to 180°C.

5. Process according to any one of Claims 1 to 4, in which the distillation step is performed in distillation apparatus comprising a distillation column, in which the pressure in said distillation column is from 1 to 1000 mbar, said column especially comprising from 2 to 40 theoretical plates.

6. Process according to any one of Claims 1 to 5, in which the mixture M1 is obtained by distillation of a mixture M'1 comprising (Rf¹-SO₂)(Rf²-SO₂)NH and Rf¹SO₂H and/or Rf²SO₂H.

7. Process according to Claim 6, in which the sulfonimide (Rf¹-SO₂)(Rf²-SO₂)NH present in the mixture M'1 is obtained via a step of acidification of an organic phase comprising a complex (Rf¹-SO₂)(Rf²-SO₂)NH,NR'₃, R' representing a linear or branched alkyl group containing from 1 to 20 carbon atoms.

8. Process according to Claim 7, in which the complex (Rf¹-SO₂)(Rf²-SO₂)NH,NR'₃ is obtained via a step of ammonolysis of Rf¹SO₂X and of Rf²SO₂X, X representing Cl, Br or F, Rf¹ and Rf² being as defined in claim 1.

9. Process according to Claim 8, in which Rf¹SO₂X is obtained via a step of oxidation of a mixture M3 comprising Rf¹CO₂M^{a}, M^{a} representing an alkali metal, and Rf¹SO₂M^{a}, to obtain a two-phase mixture M4, said mixture M4 comprising Rf¹SO₂X and Rf¹CO₂M^{a}.

10. Process according to Claim 9, in which Rf¹SO₂X, with X = F, is obtained by electrofluorination of R^{h}SO₂F, R^{h} representing a hydrocarbon-based chain identical to that of Rf¹, said chain comprising hydrogen atoms in place of the fluorine atoms, and R^{h}SO₂F optionally being obtained from R^{h}SO₂W, with W = Cl, Br or I.

11. Process according to Claim 9, in which the mixture M3 is obtained by:
- a step of sulfination of Rf¹CO₂M^{a}, and especially of CF₃CO₂K, in an organic solvent, to give a mixture M comprising Rf¹SO₂M^{a}, and especially CF₃SO₂K, and said organic solvent; and then
- a step of distillation of said organic solvent from the mixture M, to obtain the mixture M without said solvent; and then
- a step of separation of the salts of the mixture M derived from the distillation step,
where appropriate, Rf¹CO₂M^{a} being obtained via a step of salification of Rf¹CO₂H, preferably of CF₃CO₂H.

12. Process for preparing (Rf¹-SO₂)(Rf²-SO₂)NM^{b}, M^{b} representing an alkali metal, comprising
- the preparation of an aqueous solution of (Rf¹-SO₂)(Rf²-SO₂)NH according to the process as defined in Claims 1 to 11,
- a step of neutralization of (Rf¹-SO₂)(Rf²-SO₂)NH in the presence of an alkali metal base,
- where appropriate followed by a drying step.

13. Process for preparing (Rf¹-SO₂)(Rf²-SO₂)NM^{b}, comprising the following steps:
- a step a) of salification of Rf¹CO₂H to Rf¹CO₂M^{a};
- a step b) of sulfination of Rf¹CO₂Ma to Rf¹SO₂M^{a};
- a step c) of oxidation of Rf¹SO₂M^{a} to Rf¹SO₂X;
- a step d) of ammonolysis of Rf¹SO₂X to (Rf¹SO₂)(Rf²-SO₂)NH,NR'₃;
- a step e) of acidification of (Rf¹SO₂)(Rf²SO₂)NH,NR'₃ to (Rf¹SO₂)(Rf²SO₂)NH;
- a step g) of neutralization, with an alkali metal base, of (Rf¹SO₂)(Rf²SO₂)NH to (Rf¹-SO₂)(Rf²-SO₂)NM^{b}; and
- where appropriate, a step h) of drying of (Rf¹SO₂)(Rf²SO₂)NM^{b},
in which (Rf¹-SO₂)(Rf²-SO₂)NH, obtained after step e), is in the form of a mixture M1 comprising (Rf¹-SO₂)(Rf²-SO₂)NH and Rf¹SO₂H and/or Rf²SO₂H, and
i) a step of oxidizing said mixture M1 with an oxidizing agent, to obtain a mixture (Rf¹-SO₂)(Rf²-SO₂)NH is purified between step e) and step g) by performing the process as defined in Claims 1 to 11;
and M^{a} and M^{b} representing, independently of each other, an alkali metal.

14. Process for preparing (Rf¹-SO₂)(Rf²-SO₂)NM^{b}, comprising the following steps:
- a step b') of converting R^{h}SO₂W, with W representing Br, Cl or I, into R^{h}SO₂F;
- a step c') of electrofluorination of R^{h}SO₂F to Rf¹SO₂F;
- a step d) of ammonolysis of Rf¹SO₂F to (Rf¹SO₂)(Rf²-SO₂)NH,NR'₃;
- a step e) of acidification of (Rf¹SO₂)(Rf²SO₂)NH,NR'₃ to (Rf¹SO₂)(Rf²SO₂)NH;
- a step g) of neutralization, with an alkali metal base, of (Rf¹SO₂)(Rf²SO₂)NH to (Rf¹-SO₂)(Rf²-SO₂)NM^{b}; and
- where appropriate, a step h) of drying of (Rf¹SO₂)(Rf²SO₂)NM^{b},
in which (Rf¹-SO₂)(Rf²-SO₂)NH, obtained after step e), is in the form of a mixture M1 comprising (Rf¹-SO₂)(Rf²-SO₂)NH and Rf¹SO₂H and/or Rf²SO₂H, and
i) a step of oxidizing said mixture M1 with an oxidizing agent, to obtain a mixture (Rf¹-SO₂)(Rf²-SO₂)NH is purified between step e) and step g) by performing the process as defined in Claims 1 to 11;
and M^{b} representing an alkali metal, and R^{h} representing a hydrocarbon-based chain identical to that of Rf¹, said chain comprising hydrogen atoms in place of the fluorine atoms.
